# EUROPEAN PATENT APPLICATION

(11) **EP 2 072 528 A1**
(43) Date of publication of application: **24.06.2009**
(21) Application number: 07150169.6
(22) Date of filing: 19.12.2007
(51) Int. Cl.: C07K 14/47, C12N 15/85, A01K 67/027

(54) **A host cell deficient for MuRF1 and MuRF2**

(71) Applicant: Labeit, Mr. Siegfried, 69151 Neckargemünd (DE)
(72) Inventor: Labeit, Siegfried Prof. Dr., 69151 Neckargemünd (DE); Hirner, Stephanie Jutta, 68161 Mannheim (DE); Witt, Christian C., 68167 Mannheim (DE); Labeit, Dittmar, 53578 Windhagen (DE)
(74) Representative: Dick, Alexander

(57) **Abstract**

The present invention relates to a host cell deficient for a muscle ring finger 1 (MuRF1) polypeptide and a muscle ring finger 2 (MuRF2) polypeptide as well as to a non-human organism comprising said host cell. Moreover, the present invention relates to a method for identifying a gene product which is regulated by MuRF1 and MuRF2 based on determining the amount of the gene product in said host cell and said non-human organism and comparing said amount with a suitable reference amount. Also comprised by the present invention is a method for identifying a compound capable of influencing expression of a MuRF1 polypeptide and a MuRF2 polypeptide based on administering a compound to be tested to a host cell expressing the MuRF1 and the MuRF2 polypeptide, determining the amount of the MuRF1 polypeptide and the MuRF2 polypeptide, and comparing the, thus, determined amounts with a suitable reference amount. Also encompassed is a method for identifying a compound capable of influencing the biological activity of the MuRF1 and MuRF2 polypeptides as well as a compound capable of influencing interaction of the MuRF1 and MuRF2 polypeptides with an interaction polypeptide.

## Description

The present invention relates to a host cell deficient for a muscle ring finger 1 (MuRF1) polypeptide and a muscle ring finger 2 (MuRF2) polypeptide as well as to a non-human organism comprising said host cell. Moreover, the present invention relates to a method for identifying a gene product which is regulated by MuRF1 and MuRF2 based on determining the amount of the gene product in said host cell and said non-human organism and comparing said amount with a suitable reference amount. Also comprised by the present invention is a method for identifying a compound capable of influencing expression of a MuRF1 polypeptide and a MuRF2 polypeptide based on administering a compound to be tested to a host cell expressing the MuRF1 and the MuRF2 polypeptide, determining the amount of the MuRF1 polypeptide and the MuRF2 polypeptide, and comparing the, thus, determined amounts with a suitable reference amount. Also encompassed is a method for identifying a compound capable of influencing the biological activity of the MuRF1 and MuRF2 polypeptides as well as a compound capable of influencing interaction of the MuRF1 and MuRF2 polypeptides with an interaction polypeptide.

Skeletal muscle, as the largest organ in vertebrates, is important not only for motor function, but also for metabolic homeostasis. For example at rest, 10% or less of the circulatory blood flow and thus oxygen available for oxidative phosphorylation is supplied to skeletal muscles, whereas during strenuous exercise the skeletal muscles consume up to 80% of the oxygen provided by the circulatory system (Engel et al., 2004: McGraw-Hill Health Professions Division, 3rd.edit, New York). Thus, skeletal muscles constitute the major organ for the dynamic regulation of oxidative phosphorylation of energetic compounds (Pepato et al., 1996: Am J Physiol 271, E340-347). Therefore, skeletal muscle diseases such as wasting can profoundly also dysregulate metabolic body homeostasis and, vice versa, various pathological conditions such as diabetes, sepsis and cancer cachexia can cause secondary myopathies (Tiao et al., 1994: J Clin Invest 94, 2255-2264; Lecker et al., 2004: Faseb J 18, 39-51; Baracos et al., 1995: Am J Physiol 268, E996-1006; Tawa et al., 1997: J Clin Invest 100, 197-203). Myopathies developing on top of a severe metabolic disorder are increasingly encountered in clinical practice as a heterogeneous group referred to as critical illness myopathy (Solomon et al., 1996: J Biol Chem 271, 26690-26697; Latronico et al., 2005: Curr Opin Crit Care 11, 126-132). Clinically, this secondary muscle atrophy and the underlying disease states can exacerbate each other in vicious cycles, pointing towards the need for gaining a better understanding of the connections linking together metabolism and myocellular integrity in skeletal muscle. Therefore, elucidation of the molecular mechanisms causing muscle atrophies during chronic metabolic disorders such as diabetes and sepsis will be important for improving the myopathy as well as the underlying diseases (Solomon et al., 1996: J Biol Chem 271, 26690-26697; Mitch et al., 1999: Am J Physiol 276, C1132-1138; Latronico et al., 2005: Curr Opin Crit Care 11, 126-132).

Muscle atrophy is a derangement in size and number of muscle fibers occurring with aging, reduction in blood supply, or following immobilization, prolonged weightlessness, malnutrition, or after denervation. Although degradation of muscle proteins may be beneficial under certain conditions such as malnutrition, it is generally unwanted since certain pathological conditions such as longer term immobilization, chronic inflammatory states such as sepsis, chronic heart failure, diabetes, and sarcopenia during ageing, are all associated with enhanced muscle protein degradation that in turn can exacerbate the underlying disease even further.

It is assumed that the cause for muscle atrophy is a change in the balance between protein synthesis and protein degradation, and, thus the balance between anabolic and catabolic processes. Particularly, it is thought that during atrophy protein degradation pathways are activated. The particular protein degradation pathway which is thought to be responsible for loss of muscle mass is the ubiquitination of muscle proteins. By ligating at least four ubiquitin residues to muscle proteins said proteins are targeted for degradation by the proteasome.

An aim of modem medicine is to provide personalized or individualized treatment regimens. Those are treatment regimens which take into account a patient's individual needs or risks.

However, there is still no sufficient treatment for muscle wasting conditions which allows for a specific stimulation of muscle growth or muscle cell metabolism.

The striated muscle tissues and their metabolism can be stimulated by anabolic substances such as human growth hormone, insulin-like growth factor IGF-1, or steroids. Also, IGF-1 has been shown to downregulate the transcription of both atrogin-1 and MuRF1. Common side effect of these drugs is the trophic stimulation of other organ systems, including the vascular system and of connective tissue. Therefore, the long term use of these anabole stimulating drugs for treating myopathies and sarcopenia is considered as problematic.

Inhibition of calpain-Proteases, caspases, cathepsin-D, or of the ubiquitin-proteasome-system is also a potential strategy to protect muscles from wasting (for example by the proteasome inhibitor MG132 (Bonuccelli et al., 2003: Am J Pathol 163, 1663-1675). Again, this strategy is experimental and so far only tested in mice. Expected side effects are likely to include limited tissue specificity. Also, the long-term inhibition of protein degradation may lead to the accumulation of misfolded non-functional proteins and therefore will lead to cyto-toxicity. For example, the potent inhibitor of the ubiquitin/proteasome system, MG132 has cyto-toxic side effects and stimulates apoptosis (Ding et al., 2007: Apoptosis 12, 2233-2244). In the case of the proteasome inhibitor Bortezomib, cyto-toxicity is the main effect, making this drug a potent cytostatic drug for the treatment of aggressive tumors (Ding et al., 2007: Apoptosis 12, 2233-2244).

MuRF1 is a member of a gene family, comprising three distinct members, MuRF1, MuRF2, and MuRF3, all belonging to the RING finger TRIM superfamily (TRIM 63, 55, 54 respectively, Centner et al., 2001: J Mol Biol 306, 717-726). To date, is it assumed that MuRF1, MuRF2 and MuRF3 play different roles since in vivo functional characterization of MuRF1, MuRF2, and MuRF3 by mouse knock-out (KO) models and in tissue culture systems reported by different teams claimed distinct functional roles for MuRF1, MuRF2, and MuRF3, respectively.

MuRF1, when expressed in cultured cardiac myocyte cells degrades structural and regulatory muscle proteins such as troponin-1 (Kedar et al., 2004: Proc Natl Acad Sci USA 101, 18135-18140). Consistent with MuRF1's role as an anti-hypertrophy gene, aortic constrictions (that induce a chronically elevated intra-cardial pressure) caused a striking cardiac hypertrophy in a MuRF1 KO mouse model (Willis et al., 2007: Circ Res 100, 456-459). Based upon these observations, MuRF1 was proposed to function as a hypertrophy-inhibiting molecule in myocardium. No skeletal muscle phenotype in MuRF1-KO mice was reported so far in unchallenged mice.

MuRF3 is a microtuble-associated protein (Spencer et al., 2000: J Cell Biol 150, 771-784) and has an essential role for cardiac myocellular integrity as identified by mouse knock-out models: Deletion of MuRF3 causes cardiac accumulation of myosin and ruptures of the heart (Fielitz et al., 2007: Proc Natl Acad Sci USA 104, 4377-4382). This vulnerability for ruptures can be further enhanced by the additional deletion of MuRF1 (Fielitz et al., 2007: J Clin Invest 117, 2486-2495). Thus, elevated expression of MuRF1/MuRF3 might represent a cardio-protective strategy, whereas loss or inhibition of MuKF1+3 according to Fielitz and colleagues is expected to be cardio-toxic (Fielitz et al., 2007: Proc Natl Acad Sci USA 104, 4377-4382; Fielitz et al., 2007: J Clin Invest 117, 2486-2495).

MuRF2 is a distinct gene that shares high sequence similarity with MuRF1 within its aminoterminal region (for human: 88% identity for residues 1-181), whereas its carboxyterminal region is more divergent and includes domains missing in MuRF1 (Centner et al., 2001: J Mol Biol 306, 717-726). MuRF2-KO mice have been generated in an attempt to test the relationship between MuRF1 and MuRF2. So far, lack of MuRF2 activities has not been associated with muscle wasting conditions, or with another specific phenotype: MuRF2-KO mice had normal heart and skeletal muscle tissues (Willis et al., 2007: Circ Res 100, 456-459). In particular, MuRF2 appeared not to function as an antihypertrophic gene in contrast to MuRF1 (e.g. Willis et al., 2007: Circ Res 100, 456-459). Finally, tissue culture studies using expressed MuRF2 identified SQSTM1 and SRF as targets of MuRF2 (Lange et al., 2005: Science 308, 1599-1603).

There is a need for means and measures which allow for a quick and reliable identification and testing of compounds that are capable of stimulating muscle protein synthesis and for the treatment of muscle wasting conditions.

Thus, the technical problem underlying the present invention must be seen as the provision of means and methods for complying with the aforementioned needs.

The technical problem is solved by the embodiments characterized in the claims and herein below.

Accordingly, the present invention relates to a host cell that is deficient for a muscle ring finger 1 (MuRF1) polypeptide and for a muscle ring finger 2 (MuRF2) polypeptide.

MuRF1 (muscle ring finger 1) and MuRF2 (muscle ring finger 2) belong to a small family of muscle ring finger binding proteins (MuRF1, MuRF2 and MuRF3, also known as RNF 28, 29, and 30) and are shown to associate with myofibrils and/or microtubuli. Structurally, the three known MURFs are members of the RING finger-B box-coiled-coiled (RBCC) family, a group of proteins implicated in ubiquitination and signal transduction. All three MURF members can homo- and hetero-oligomerize, apparently via their coiled coil domains, although the functional implications of their oligomerization are not known. MURF1 is a titin-binding protein, specifically interacting with the C-terminal immunoglobulin (Ig) domains, A168-A170, adjacent to the titin kinase domain (Centner et al., 2001: J Mol Biol 306, 717-726). MURF1 also binds the small ubiquitin-related modifier-3 (SLTMO-3/SMT3b) and the glucocorticoid modulatory element binding protein-1 (GMEB-1).

The amino acid and nucleic acid sequences of MuRF1 and MuRF2 of various organisms are well known in the art. MuRF1 is well conserved throughout vertebrates, particularly throughout mammals (conservation of human MuRF1 peptide to dog is 91%; to mouse and rat is 83%; to Xenopus laevis is 65%). E.g, MuRF1 is well described in humans, mice, rats. MuRF2 is less conserved in the animal kingdom and moreover, occurs in several splice isoforms (Centner et al., 2001: J Mol Biol 306, 717-726; Pizon et al., 2002: J Cell Sci 115, 4469-4482; McElhinny et al., 2004: J Cell Sci. 117, 3175-3188). When restricting comparisons to the aminoterminal region comprising residues 1-315 that is common to the different MuRF2 isoforms, avian MuRF2 shares 76% homology to human MuRF2, and mouse MuRF2 shares 83% homology with human MuRF2.The sequences of mouse MuRF1 is preferably described in (Bodine et al., 2001: Science 294, 1704-1708), and for MuRF2 is preferably, described in (Strausberg et al., 2002: Proc Natl Acad Sci USA 99, 16899-16903; GenBank accession number, reporting BC153052.1). The amino acid sequences of human MuRF1 and MuRF2 are shown in SEQ ID NO:1 and SEQ ID NO:2, respectively. The nucleic acid sequences (cDNAs) encoding said polypeptides are shown in Seq ID No:3 and SEQ ID NO:4, respectively (see also Centner et al., 2001: J Mol Biol 306, 717-26, reporting AJ291712.1 and AJ291713.1). Preferably, MuRF1 polypeptide or MuRF2 polypeptide as used herein relates to a polypeptide which is at least 70%, 80%, 90%, 95%, 96%, 97%, 98%, 99% or 100% identical to the polypeptide as shown in SEQ ID NO:1 and SEQ ID NO:2, respectively. Preferably, MuRF1 nucleic acid or MuRF2 nucleic acid as used herein relates to a nucleic acid which is at least 70%, 80%, 90%, 95%, 96%, 97%, 98%, 99% or 100% identical to the nucleic acid as shown in SEQ ID NO:3 and SEQ ID NO:4, respectively. The degree of identity between two sequences can be determined by algorithms well known in the art. Preferably, the degree of identity is to be determined by comparing two optimally aligned sequences over a comparison window, where the fragment of amino acid sequence in the comparison window may comprise additions or deletions (e.g., gaps or overhangs) as compared to the reference sequence (which does not comprise additions or deletions) for optimal alignment. The percentage is calculated by determining the number of positions at which the identical amino acid residue occurs in both sequences to yield the number of matched positions, dividing the number of matched positions by the total number of positions in the window of comparison and multiplying the result by 100 to yield the percentage of sequence identity. Optimal alignment of sequences for comparison may be conducted by the local homology algorithm (Smith TF and Waterman MS, 1981: J Mol Biol. 147, 195-197), by the homology alignment algorithm (Needleman and Wunsch, 1970: J Mol. Biol. 48, 443-453), by the search for similarity method (Pearson and Lipman, 1988: Proc. Natl. Acad Sci. (USA) 85, 2444-2448), by computerized implementations of these algorithms (GAP, BESTFIT, BLAST, PASTA, and TFASTA in the Wisconsin Genetics Software Package, Genetics Computer Group (GCG), 575 Science Dr., Madison, WI), or by visual inspection. Given that two sequences have been identified for comparison, GAP and BESTFIT are preferably employed to determine their optimal alignment and, thus, the degree of identity. Preferably, the default values of 5.00 for gap weight and 0.30 for gap weight length are used.

The term "host cell" in the context of the present invention, preferably, relates to a eukaryotic host cell, more preferably to a vertebrate host cell and, even more preferably, to a mammalian host cell, and most preferably a rodent host cell. Rodent host cells, preferably, include mouse cells, rat cells and hamster cells. Preferably, the host cell is a non-human cell. It is to be understood that the host cell of the present invention and, thus, a host cell deficient for a MuRF1 and MuRF2 polypeptide is derived from a host cell that originally comprised a functional MuRF1 and MuRF2 polypeptide. Preferably, said host cell is a muscle cell, more preferably a striated muscle cell, even more preferably a myocardial muscle cell and, most preferably, a skeletal muscle cell.

The term "deficient for" means that a host cell does not exhibit significant MuRF1 and MuRF2 polypeptide activity. This can be, preferably, achieved by providing a host cell that does not express the MuRF1 and MuRF2 polypeptide by methods described elsewhere herein. Such host cell, preferably, does not contain MuRF1 or MuRF2 polypeptides at all. Also contemplated by the present invention is a host cell in which the amount of the MuRF1 and MuRF2 polypeptides is significantly reduced compared with a wild-type host cell, thus, compared with a host cell of the same species that comprises the MuRF1 and MuRF2 polypeptide and which, thus, is not deficient for the MuRF1 and MuRF2 polypeptide. Preferably, the amount of the MuRF1 and MuRF2 polypeptide in a host cell of the present invention is reduced by 30 %, 40%, 50%, 60%, 70%, 80%, 90%, 95%, more preferably by 99% and, most preferably, by 100%. Activity is measured by methods well known in the art (see, e.g., Examples).

Preferably, the host cell deficient for the MuRF1 and MuRF2 polypeptide is prepared by knocking out the MuRF1 and MuRF2 polypeptide and, thus, is a MuRF1/MuRF2 double knock out. A "Knock out" in the context of the present invention, preferably, refers to a host cell or non human organism comprising a targeted null-mutation of the gene function of the MuRF1 and MuRF2 polypeptide, and thus, to a host cell or an organism in which the endogenous genes/polypeptides for MuRF1 and MuRF2 are made non-functional. It is well known in the art how to prepare a knock-out organism, particularly a knock-out mouse.

A host cell deficient for the MuRF1 and MuRF2 polypeptide, preferably, may also be generated by other methods well known in the art. Preferably, said host cell is generated via an RNAi approach, e.g. by expressing or administering double stranded RNA molecules which specifically target the MuRF1 and MuRF2 transcripts leading to a significantly decreased expression of the MuRF1 and MuRF2 polypeptide. Moreover, it is also contemplated that the host cell deficient for the MuRF1 and MuRF2 polypeptide is generated by expressing antisense RNA molecules that specifically target polynucleotides encoding for the MuRF1 and MuRF2 polypeptide. Moreover, a host cell that is deficient for the MuRF1 and MuRF2 polypeptides can be generated by introducing triple helix molecules that specifically target the polynucleotides encoding the MuRF1 and MuRF2 polypeptides. It is known in the art that triplex-forming oligonucleotides that can bind to duplex DNA in a sequence-specific manner are tools to achieve targeted gene modification. Also encompassed by the invention that the host being deficient for both the MuRF1 and MuRF2 polypeptide is generated by introducing ribozymes which are capable of targeting MuRF1 and MuRF2 into said host cells. A ribozyme is a RNA which is capable of specifically interacting with a target RNA and of irreversibly cleaving it at a defined site.

It is to be understood that a host cell that is deficient for the MuRF1 and MuRF2 polypeptide also can be a host cell comprising incomplete MuRF1 and MuRF2 polypeptides, thus MuRF1 and MuRF2 polypeptides lacking certain parts or a certain part.

Preferably, said parts are parts of the MuRF1 and MuRF2 polypeptide that are responsible for the biological activity of the MuRF1 and MuRF2 polypeptide, respectively. These parts are frequently also referred to as functional domains. Preferred functional domains of the MuRF1 and MuRF2 are the polypeptide regions conferring to the enzymatic activity of the MuRF1 and MuRF2 polypeptide. Since it is well known in the art that the MuRF1 and MuRF2 polypeptide have E3 ubiquitin-protein ligase activity (herein also referred to as ligase activity), preferred domains that may not be present in a host cell being deficient for the MuRF1 and MuRF2 polypeptides are domains contributing to the ligase activity of MuRF1 and MuRF2, thus domains that ubiquitinate or sumoylate the substrate(s) of MuRF1 and MuRF2. Said domains are preferably the RING finger domains in MuRF1 and MuRF2, comprising residues 1-66 in CAC32840.1 and CAC33173.1, and, thus, of the sequence shown SEQ ID NO:1 and 2, respectively. Moreover, functional domains may be determined by in silico analysis. Functional domains, preferably, also encompass domains that allow binding to an interaction polypeptide or recognizing an interaction polypeptide of the MuRF1 and MuRF2 polypeptide. The term "interaction polypeptide" is described elsewhere in the specification.

Also contemplated is that the host cell deficient for the MuRF1 and MuRF2 polypeptide comprises instead of wild-type MuRF1 and MuRF2 polypeptides modified (and thus mutated) MuRF1 and MuRF2 polypeptides provided that the mutated MuRF1 and MuRF2 polypeptides have a reduced biological activity compared to the wild-type MuRF1 and MuRF2 polypeptides. Preferably, said modified polypeptides are generated by substituting, adding or deleting at least one amino acid by methods well known in the art.

Moreover, the present invention also relates to a non-human organism comprising a host cell according to the invention, i.e. a host cell deficient for the MuRF1 and MuRF2 polypeptide. Preferably, said non-human organism is a mammal, more preferably a rodent, even more preferably, a rat and, most preferably, a mouse. In a preferred embodiment said non-human organism is a MuRF1/MuRF2 double-knock-out mouse (homozygous for the knock-outs).

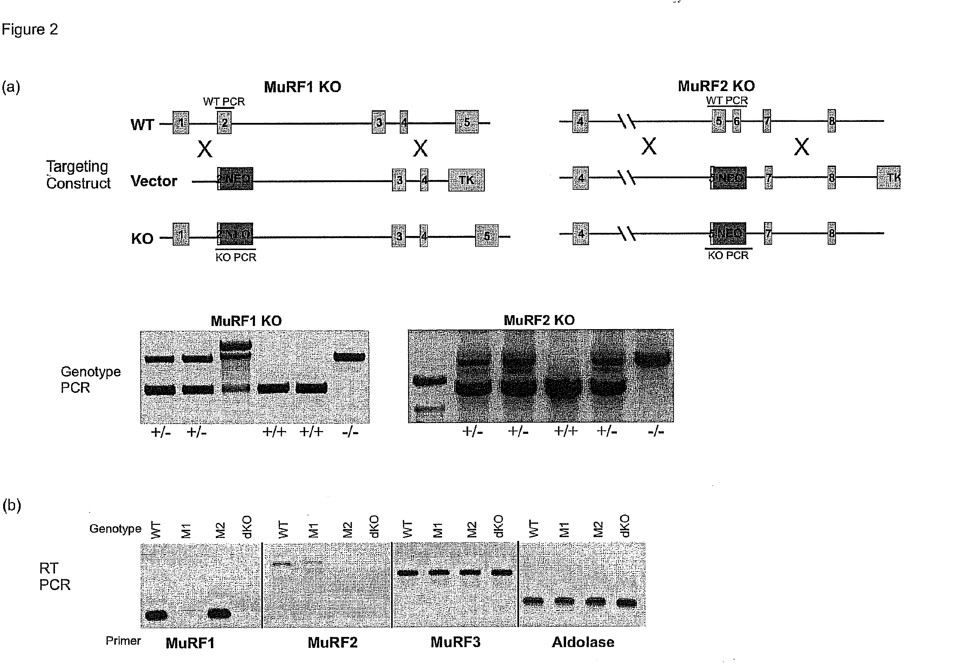

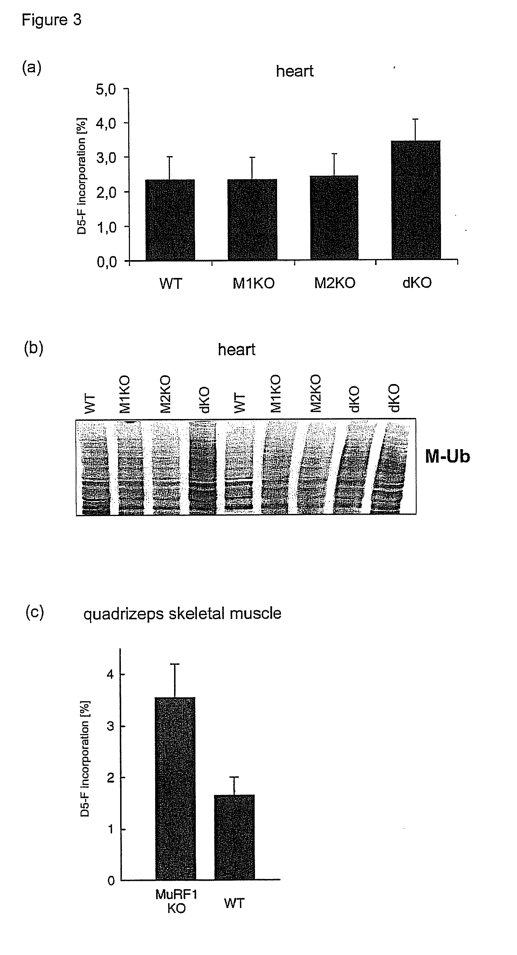

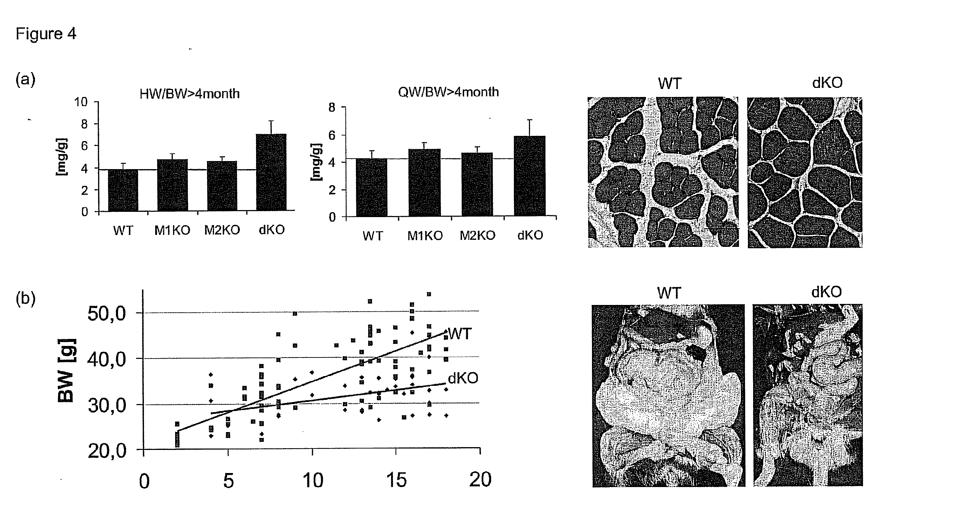

Surprisingly, it was shown in the studies underlying the present invention that a host cell or organism which is deficient for both the MuRF1 and the MuRF2 polypeptide (see Figure 2) showed a stimulated muscle metabolism (Figure 3). Specifically, it was shown that de novo muscle protein synthesis is significantly increased in MuRF1/MuRF2 double knock-out (dKO) mice (Figure 3). Consistent with a highly synergistic and combined action of MuRF1 and MuRF2 on muscle, both muscle mass and myofiber diameter responded only to the combined inactivation of MuRF1 and MuRF2 (Figure 4a). Moreover, and even more surprising, the combined inactivation of MuRF1 and MuRF2 prevented the accumulation of body fat whereas wild-type control mice (thus mice being not deficient for MuRF1 and MuRF2) showed an accumulation of body fat (Figure 4b). The combined inactivation of MuRF1 and MuRF2 is compatible with postnatal longevity (Figure 4b). Mice that were deficient for either MuRF1 or MuRF2 showed a negligible effect on de novo synthesis of muscle proteins (Figure 4a). Moreover, the studies underlying the present invention clearly show that neither MuRF1, MuRF2 nor MuRF1/MuRF2 double knock out mice have reduced muscle protein degradation nor reduced levels of multi-ubiquitinated muscle proteins (Figure 3b). Instead, an enhanced de-novo synthesis of muscle proteins was detected in MuRF1/MuRF2 double knock out mice. These data demonstrating the striking synergistic action on muscle only after inactivation of both MuRF1 and MuRF2 are surprising since so far, only MuRF1 has been implicated to mediate muscle atrophy as an atrogin-lilce factor.

Advantageously, it was shown that the stimulated muscle protein synthesis is coupled to an enhanced glucose and lipid metabolism. In particular, lipids are efficiently metabolized in MuRF1/MuRF2 double KO animals. Thus, the phenotype of MuRF1/MuRF2 double KO mice shows that the combined inactivation of MuRF1 and MuRF2 results in global and potent stimulatory effects on muscle metabolism.

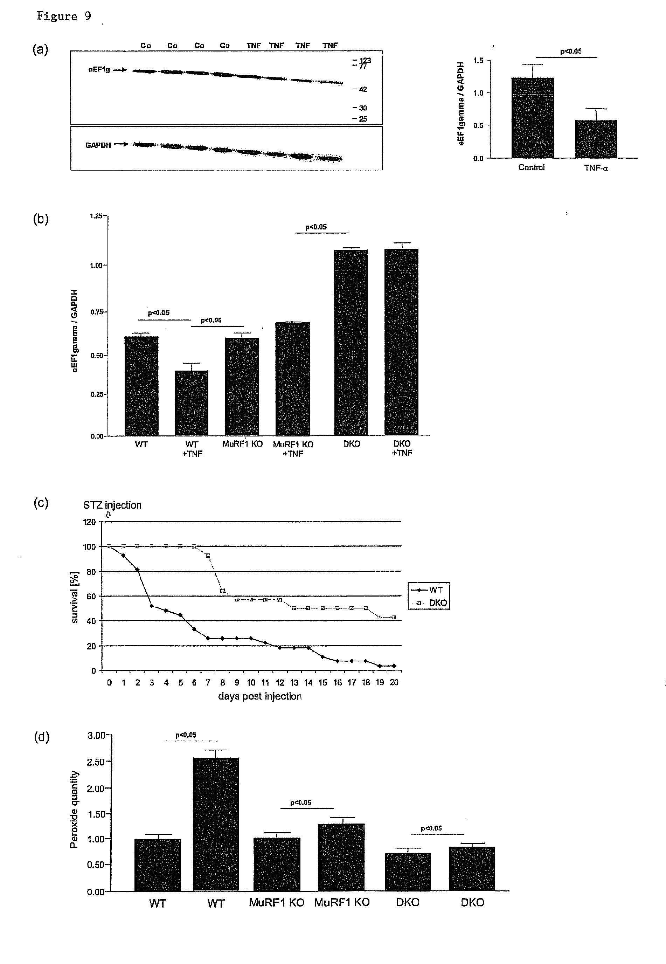

Surprisingly, it was shown that the combined inactivation of MuRF1 and MuRF2 also protects from cell damage, specifically from tumor necrosis factor-alpha (TNF-alpha) and streptocozin (STZ) induced cell damage, both toxins that induce apoptosis, oxidative stress, and impair muscle protein synthesis. Intriguingly, MuRF1/MuRF2 dKO mice show a high resistance to tumor necrosis factor alpha (TNF-alpha) and streptozocin (STZ) toxicity (Figure 9). The mechanisms include preservation of sufficient levels of translation factors (Figure 9b), and protection from the generation of reactive oxygen species (ROS; see Figure 9d), together causing at least in part a much higher survival in MuRF1/MuRF2 KO mice when injecting lethal doses of STZ (Figure 9c). This combination of protective effects of MuRF1 and MuRF2 inhibitors will be advantageous in chronic cellular stress situations such as long term ischemia, diabetes, and sepsis, all linked to elevated inflammatory cytokine levels, and apoptosis.

The present invention will be; if applied, very beneficial, since the combined inhibition of both the MuRF1 and MuRF2 polypeptide allows to prevent, to treat or to ameliorate conditions which are linked to reduced synthesis of muscle proteins such as ageing or long term immobilization, both conditions leading to sarcopenia because of an impaired balance of degradation and de novo protein synthesis. Similarly, systemic stress situations, such as sepsis and diabetes impair muscle protein synthesis and at the same time increase toxic reactive oxygen species (ROS), causing thereby myocellular damage and apoptosis. The protection of MuRF1/MuRF2 dKO mice after induction of ROS signalling by TNF-alpha will be beneficial to prevent myocellular damage during chronic stress situations (see Figure 9). The combined effects on muscle metabolism and cellular protection will be in particular beneficial when treating the metabolic syndrome (MS), where muscle metabolism is reduced, oxidative stress increased at the same time. Finally, MS is frequently linked with pathological adipositas. Inactivation of MuRF1 and MuRF2 leads to a longterm dramatic loss of body fat (Figure 3b), thus presenting another desired effect. The clinical syndromes of the aforementioned diseases, disorders and conditions are well known in the art.

Taken together, based upon our phenotypic data of MuRF1/MuRF2 dKO mice, inhibition of MuRF1 and MuRF2 will be beneficial for the treatment of muscle wasting conditions, particularly sarcopenia, decrease of muscle mass due to sepsis, decrease of muscle mass due to diabetes. Furthermore, it will be beneficial for the treatment of adipositas. Moreover, a compound identified by the methods of the present invention that inhibits both MuRF1 and MuRF2 will be valuable (e.g. due to its effect on the muscle, glucose and lipid metabolism) for the treatment, amelioration and prevention of diabetes, chronic heart failure, metabolic syndrome and insulin resistance and myocellular damage (particularly, when linked to metabolic disorders, including pathological TNF-alpha/ROS signalling).

Moreover, the present invention relates to a method for identifying a gene product which is regulated by a MuRF1 polypeptide and a MuRF2 polypeptide, comprising the steps of
a) determining the amount of a gene product, suspected to be regulated by a MuRF1 and a MuRF2 polypeptide, in the host cell which is deficient for the MuRF1 and MuRF2 polypeptide and/or in a sample obtained from the non-human organism which is deficient for the MuRF1 and MuRF2 polypeptide, and
b) comparing the amount as determined in step a) with a suitable reference amount.

The aforementioned method according to the invention is a screening method. Preferably, said method in an in vitro method.

The term "gene product" as used herein, preferably, relates to any biological product or product produced as a result of the biochemical reactions that occur under the control of the MuRF1 and MuRF2 polypeptides. Preferably, a gene product in the context of the present invention is a polypeptide or an RNA molecule such as a messenger RNA molecule. The terms "polypeptide" and "protein" are used interchangeably throughout the application. A "gene product", preferably, also relates to a post-translationally modified polypeptide. Post-translational modifications are, preferably, chemical modifications of a polypeptide during or after its translation. Post-translational modifications are well known in the art. Preferred post-translational modifications are ubiquitination, sumoylation, phosphorylation such as phosphorylation of a serine, threonine and/or tyrosine residue, glycosylation, prenylation, myristoylation, farnesylation, attachment of a fatty acid, attachment of a GPI anchor, acetylation, methylation and nucleotidylation such as ADP-ribosylation. The most preferred post-translational modifications are ubiquitination and sumoylation which are described elsewhere in this specification. The term "gene product", preferably, also relates to alternatively spliced RNA molecules.

As used herein, the term "identifying", preferably, relates to determining which gene products or which gene products, preferably, out of a pool of one or more gene products that are tested is regulated by the MuRF1 and MuRF2 protein.

As used herein the term "regulated by" means that the amount of a gene product is modulated, and thus, increased or decreased by the MuRF1 and MuRF2 polypeptide in combination. Said modulation, preferably, may be directly or indirectly.

Determining the amount of a gene product, and, thus, of the amount of an RNA, a polypeptide or a post-translationally modified polypeptide relates to measuring the amount or concentration, preferably semi-quantitatively or quantitatively. Measuring can be done directly or indirectly. Direct measuring or a polypeptide or a post-translationally modified polypeptide, preferably, relates to measuring the amount or concentration of the polypeptide based on a signal which is obtained from the polypeptide itself and the intensity of which directly correlates with the number of molecules of the polypeptide present in the sample. Such a signal - sometimes referred to herein as intensity signal -may be obtained, e.g., by measuring an intensity value of a specific physical or chemical property of the polypeptide. Indirect measuring includes measuring of a signal obtained from a secondary component (i.e. a component not polypeptide itself) or a biological read out system, e.g., measurable cellular responses, ligands, labels, or enzymatic reaction products.

In accordance with the present invention, determining the amount of a peptide or polypeptide can be achieved by all known means for determining the amount of a peptide in a sample. Said means comprise immunoassay devices and methods that may utilize labelled molecules in various sandwich, competition, or other assay formats. Said assays will develop a signal which is indicative for the presence or absence of the peptide or polypeptide. Moreover, the signal strength can, preferably, be correlated directly or indirectly (e.g. reverse- proportional) to the amount of polypeptide present in a sample.

Further suitable methods comprise measuring a physical or chemical property specific for the peptide or polypeptide such as its precise molecular mass or NMR spectrum. Said methods comprise, preferably, biosensors, optical devices coupled to immunoassays, biochips, analytical devices such as mass- spectrometers, NMR- analyzers, or chromatography devices. Further, methods include micro-plate ELISA-based methods, fully-automated or robotic immunoassays (available for example on Elecsys^{™} analyzers), CBA (an enzymatic Cobalt Binding Assay, available for example on Roche-Hitachi^{™} analyzers), and latex agglutination assays (available for example on Roche-Hitachi^{™} analyzers).

Methods to determine the amount of a specific RNA in a sample are well known in the art. Preferred methods are PCR, Northern Blotting and microarray analysis.

It is also contemplated to determine the amount of a gene product by operably linking the gene product or the regulatory elements conferring expression of said gene product (frequently also referred to as promoter) to a suitable reporter. Preferably, if the gene product is mRNA, the regulatory elements conferring expression of said mRNA are linked to a polynucleotide encoding a reporter gene. A polynucleotide encoding a reporter gene, preferably, is a polynucleotide that encodes a molecule that can be detected readily, either directly or by its effect on the host cell. A number of reporter genes are known in the art such as β-galactosidase, luminescent or fluorescent proteins, such as Green Fluorescent Protein (GFP) and variants thereof. Preferably, also a polypeptide can be operably plinked to a reporter protein by well known methods such as translational fusion.

The term "amount" as used herein encompasses the absolute amount of a gene product, and thus of a RNA, of a polypeptide or a post-translationally modified polypeptide as well as any value or parameter which correlates thereto or can be derived therefrom. Such values or parameters comprise intensity signal values from all specific physical or chemical properties obtained from the said gene product, e.g. if the gene product is a polypeptide intensity values in mass spectra or NMR spectra. Moreover, with respect to polypeptides encompassed are all values or parameters which are obtained by indirect measurements specified elsewhere in this description, e.g., response levels determined from biological read out systems in response to the polypeptides or intensity signals obtained from specifically bound ligands. It is to be understood that values correlating to the aforementioned amounts or parameters can also be obtained by all standard mathematical operations.

The term "comparing" as used herein encompasses comparing the amount of a gene product, and thus of a RNA, a polypeptide or a post-translationally modified polypeptide comprised by the sample to be analyzed with an amount of a suitable reference source specified elsewhere in this description. It is to be understood that comparing as used herein refers to a comparison of corresponding parameters or values, e.g., an absolute amount is compared to an absolute reference amount while a concentration is compared to a reference concentration or an intensity signal obtained from a test sample is compared to the same type of intensity signal of a reference sample. The comparison referred to in step (b) of the method of the present invention may be carried out manually or computer assisted. For a computer assisted comparison, the value of the determined amount may be compared to values corresponding to suitable references which are stored in a database by a computer program. The computer program may further evaluate the result of the comparison, i.e. automatically provide the desired assessment in a suitable output format. Based on the comparison of the amount determined in step a) and the reference amount, it is possible to assess whether a gene product is regulated by MuRF1 and MuRF2.

Accordingly, the term "reference amount" in the context with the aforementioned method, preferably, refers to an amount which allows assessing whether a gene product is regulated by MuRF1 and MuRF2 as referred to above. The person skilled in the art is familiar with selecting a suitable reference amount. Preferably, the reference is be derived from a corresponding host cell or a non-human organism that is not deficient for the MuRF1 and MuRF2 polypeptide. Furthermore, the reference amount may be derived from a host cell or a sample of a non-human organism that is deficient for either the MuRF1 polypeptide or the MuRF2 polypeptide. Moreover, the reference amount may defme a threshold amount, whereby an amount larger or lower than the threshold shall be indicative for gene product regulated by the MuRF1 and MuRF2 polypeptide. The reference amount applicable for an individual gene product may vary depending on various parameters such as growth conditions. A suitable reference amount may be determined by the method of the present invention from a reference sample to be analyzed together, i.e. simultaneously or subsequently, with the test sample.

Preferably, the reference amount as referred to above is an amount of the gene product in a host cell and/or in a sample of an organism which is not deficient for the MuRF1 polypeptide and the MuRF2 polypeptide (such as a wild-type host cell or organism). Preferably, a change of the amount as determined in the aforementioned method indicates that the tested gene product is regulated by the MuRF1 polypeptide and the MuRF2 polypeptide.

A change in the context of the present invention, preferably, is a more than 1.5 fold, 2.0 fold, 3.0 fold, 4.0 fold, 5.0 fold, 7.0 fold, 10.0 fold, 20.0 fold or 50.0 fold change and, thus, a 1.5 fold, 2.0 fold, 3.0 fold, 4.0 fold, 5.0 fold, 7.0 fold, 10.0 fold, 20.0 fold or 50.0 increase, or, more preferably, decrease of the amount of the tested gene product compared with the corresponding reference.

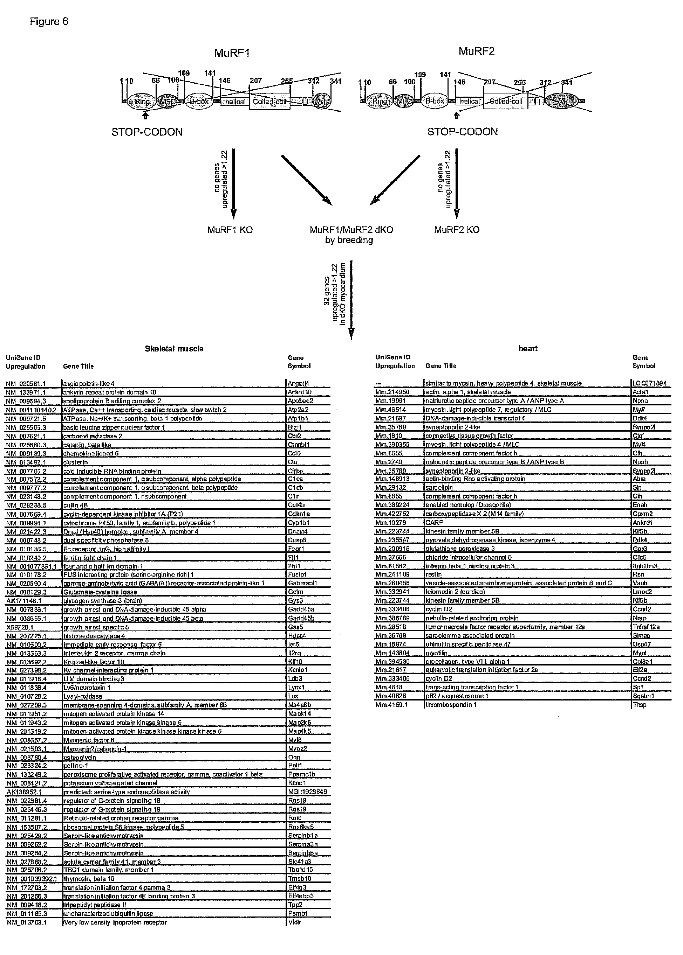

Advantageously, it was shown that the transcriptome in muscle tissue of MuRF1/MuRF2 double knock out mice (and thus of an organism being deficient of the MuRF1 and MuRF2 polypeptide) significantly differs from the transcriptome in muscle tissues of mice which are not deficient for MuRF1 and MuRF2 or which are only deficient for either the MuRF1 or the MuRF2 polypeptide. An overview on gene products that are regulated by both MuRF1 and MuRF2 can be found in the table of Fig. 6.

Thus, by applying the aforementioned method of the present invention the identification of gene products or pathways with are regulated by MuRF1 and MuRF2 will be identified. The, thus, identified gene products represent valuable targets for the treatment or prevention of myocellular damage and wasting conditions such as myopathies during diabetes and sepsis, and where muscle protein synthesis is chronically reduced, such as sarcopenia during ageing. Moreover, the identified gene products will be promising candidate targets for the treatment of adipositas, because of the loss of body fat observed in dKO mice.

Moreover, the present invention also relates to a method for identifying a compound that influences the expression, function and or biological activity of a gene product identified by the aforementioned method, thus of a gene product which is regulated by both MuRF1 and MuRF2. Particularly preferred gene products in this context which are regulated by both MuRF1 and MuRF2 are listed in the tables of Fig. 6. The gene products listed in the the table will be promising candidates for treating and preventing and ameliorating diseases, disorders and conditions referred to herein. Compounds influencing the expression, function and/or biological activity of said gene products can be identified by carrying out methods as described herein elsewhere. Moreover, the present invention relates to the use of a gene product shown in the tables of Fig. 6 as a marker for a disease, condition or disorder referred to above.

In a preferred embodiment the amount of the gene product suspected to be regulated by the MuRF1 and MuRF2 polypeptide is additionally determined in (i) a host cell or non-human organism which is deficient for the MuRF1 polypeptide but not for the MuRF2 polypeptide (preferably, a MuRF1 knock-out) and/or (ii) a host cell or non-human organism which is deficient for the MuRF2 polypeptide but not for the MuRF1 polypeptide (preferably, a MuRF2 knock-out). The, thus, determined amounts, preferably, are then compared with at least one suitable reference amount which, is preferably the amount of said gene product in a host cell or organism which is not deficient for MuRF1 (for (i)) and/or MuRF2 (for (ii)).

The definitions made with regard to the aforementioned method apply *mutatis mutandis* to the following method, unless otherwise stated.

Moreover, the present invention relates to a method for identifying a compound capable of influencing expression of a MuRF1 polypeptide and a MuRF2 polypeptide, comprising the steps,
a) administering a test compound to a host cell or a non-human organism expressing the MuRF1 and the MuRF2 polypeptide
b) determining the amount of the MuRF1 polypeptide and the MuRF2 polypeptide, and
c) comparing the amounts as determined in step b) with a suitable reference amount (thus a reference amount for MuRF1 and a reference amount for MuRF2),
wherein a change of the amount of the MuRF1 polypeptide and the MuRF2 polypeptide as determined in step b) compared with the reference amount indicates that the tested compound is capable of influencing expression of the MuRF1 polypeptide and the MuRF2 polypeptide.

The aforementioned method of the present invention is a screening method. Said method may be carried out in vivo, in vitro or ex vivo. The term "in vivo", "in vitro" and "ex vivo" are well known in the art. "In vivo" as used in the context of the method of present invention, preferably, relates to experiments done in or on the living body of a non human organism, such as animal tests. The term "in vitro", preferably, relates to experiments in cells that are cultured outside of an organism, such as tissue culture experiments. The term "ex vivo", preferably, refers to experiments in which cells are removed from a living organism and are then cultured outside the organism.

The term "expressing the MuRF1 and MuRF2 polypeptide" is understood by the skilled person. The term "expressing", preferably, relates to the biosynthesis of an RNA molecule and/or a polypeptide through endogenous cellular mechanisms. The term, preferably, includes transcription, translation, and post-translational modification of MuRF1 and MuRF2.

A host cell or a non-human organism expressing MuRF1 and MuRF2 may be any host cell or non-human that naturally comprises the MuRF1 or MuRF2 polypeptide (or fragments or variants thereof). A host cell in the context of the aforementioned method, preferably, is a vertebrate cell, more preferably, a mammalian cell, even more preferably a rodent cell, and, most preferably a rat or a mouse cell. Preferably, said host cell is non-human. Cells that are particularly useful in the context of the present invention are striated muscle cells, particularly cardiac muscle cells and skeletal muscle cells. Accordingly, the non-human organism expressing the MuRF1 and MuRF2 polypeptide is preferably a vertebrate, more preferably a mammal, even more preferably a rodent and most preferably a rat or a mouse.

Preferably, the non-human organism will be sacrificed after the method has been carried out. It is to be understood that such an in vivo screening method is not deemed to be a method of treatment of the human or animal body.

By applying the aforementioned method compounds can be identified that are capable of influencing MuRF1 and MuRF2 expression. Preferably, the identified compounds inhibit the expression of the MuRF1 and MuRF2 polypeptide. Compounds that are identified by said method will be, preferably, promising candidates for the stimulation of de novo muscle protein synthesis and inhibition of ROS-signalling, and thus, for the prevention or treatment of muscle wasting and myocellular damage during sepsis, diabetes, ageing, and chronic heart failure. The stimulated muscle metabolism is also a suitable strategy to combat increased body fat and inactive skeletal muscle tissues and thus, for the treatment or prevention of conditions, disorders and diseases mentioned herein, preferably of muscle wasting conditions and for the metabolic syndrome. Thus, the aforementioned method also relates to a method for identifying a compound capable for the treatment and prevention of the aforementioned conditions, disorders and diseases.

A compound to be tested can be, preferably, any molecule deemed appropriate. Preferred compounds to be tested encompass proteins, peptides, drugs, small molecules and small organic molecules. A compound to be tested may be, e.g., derived from a compound library comprising natural and/or synthetic compounds of which a large number is known in the art.

As used herein, the term "identifying", preferably, relates to determining which compound or which compounds, preferably, out of a pool of one or more compounds that are tested is capable of influencing MuRF1 and MuRF2 expression. It is to be understood that depending on the tested compounds none of the tested compounds may be capable of influencing MuRF1 and MuRF2 expression.

It is also contemplated that the tested compound is a compound that already has been identified by other methods as being capable of influencing MuRF1 and MuRF2 expression. The compound may be also a compound that was shown to stimulate muscle growth or being suitable for the treatment of sarcopenia. Thus, the method of the present invention also allows confirmation of an effect of a compound.

As mentioned above, the compound identified by the aforementioned method is a compound that is capable of influencing the expression of the MuRF1 and MuRF2 polypeptide. As used herein the term "influencing expression of the MuRF1 and MuRF2 polypeptide" means that the amount of the MuRF1 and MuRF2 polypeptide in a host cell or a non-human organism expressing MuRF1 and MuRF2 is modulated and, thus, changed by said compound. The term "influencing", preferably, means enhancing the expression (and thus the amounts) of the MuRF1 and MuRF2 polypeptide and, more preferably, reducing, and, thus, inhibiting/decreasing the expression (and thus the amounts) of the MuRF1 and MuRF2 polypeptide. It is to be understood that a compound that influences expression of the MuRF1 and MuRF2 polypeptide may influence transcription of the polynucleotides encoding the MuRF1 and MuRF2 polypeptides into an RNA molecule, the splicing of the MuRF1 and MuRF2 RNA molecules, translation of MuRF1 and MuRF2 mRNA into the MuRF1 and MuRF2 polypeptide, and posttranslational modification of the MuRF1 and MuRF2 polypeptides and, thus, the amount thereof. How to determine the amount RNA molecules and polypeptides is described elsewhere herein. As already described herein, the expression of MuRF1 and MuRF2 may be assessed by determining the amount of a suitable reporter gene that, e.g., is operably linked to the promoter regions of polynucleotides encoding the MuRF1 and MuRF2 polynucleotides and/or that is translationally fused with the MuRF1 and MuRF2 polypeptides.

Administering a test compound, preferably, refers to delivery of a compound to be tested to a host cell or a non-human organism expressing the MuRF1 and MuRF2 polypeptide. The administration can be done by any method deemed appropriate. It is to be understood that the administration of the test compound may depend on whether the identification of a compound capable of influencing the expression of the MuRF1 and MuRF2 polypeptide is done in vivo, in vitro or in ex vivo. Preferably, when the testing is done in vivo, the compound to be tested is administered orally, parenterally or rectally. Parenteral administration, preferably, includes cutaneous, subcutaneous, intranasal, vaginally, intravenous, and, most preferably, intramuscular administration. It is to be understood that the amount of the compound to be tested may differ, since the effect of a compound that is capable of influencing expression of the MuRF1 and MuRF2 polypeptide may depend on the amount that is administrated. Thus, the method of the present invention also allows the determination of a therapeutically effective amount of a tested compound.

A suitable reference amount in the context of the aforementioned method is, preferably, derived from a corresponding host cell or in a corresponding sample of a non-human organism expressing the MuRF1 and MuRF2 polypeptide to which the tested compound has not been administered. Also contemplated by the present invention is that the reference amount is derived from a host cell or organism to which a different, preferably, a lower amount of the compound to be tested was administered.

As mentioned above a change of the amount of the MuRF1 polypeptide and the MuRF2 polypeptide as determined compared with a reference amount indicates that the tested compound is capable of influencing expression of the MuRF1 polypeptide and the MuRF2 polypeptide. Preferably, such change is a statistically significant change. More preferably, said change is a more than 1.5 fold, 2.0 fold, 3.0 fold, 4.0 fold, 5.0 fold, 7.0 fold, 10.0 fold, 20.0 fold or 50.0 fold change and, thus, a 1.5 fold, 2.0 fold, 3.0 fold, 4.0 fold, 5.0 fold, 7.0 fold, 10.0 fold, 20.0 fold or 50.0 increase or decrease compared with the reference amount. Preferably, a decrease of the amount of the MuRF1 polypeptide and the MuRF2 polypeptide in the host cell or organism to which the tested compound has been administered compared with the reference amount indicates that the tested compound is capable of inhibiting expression of the MuRF1 polypeptide and the MuRF2 polypeptide and, therefore. Preferably, said compound is capable for the treatment of diseases, disorders and conditions as described herein.

Moreover, the present invention relates to method for identifying a compound capable of influencing the biological activity of a MuRF1 polypeptide and a MuRF2 polypeptide, comprising the steps,
a) administering a test compound to a host cell or a non-human organism expressing the MuRF1 and the MuRF2 polypeptide
b) determining the biological activity of the MuRF1 polypeptide and the MuRF2 polypeptide, and
c) comparing the biological activity as determined in step b) with a suitable reference biological activity (thus a reference for the MuRF1 and a reference for the MuRF2 polypeptide),
wherein a change, preferably a decrease, of the biological activity of the MuRF1 polypeptide and the MuRF2 polypeptide as determined in step b) compared with the reference biological activity indicates that the tested compound is capable of influencing the biological activity of the MuRF1 polypeptide and the MuRF2 polypeptide.

The terms "host cell or non-human organism expressing the MuRF1 and MuRF2 polypeptide", "identifying" "compound" "administering a test compound", "determining", "comparing", and "reference" as well as suitable references have been defined elsewhere in this specification. It is to be understood that the term "comparing" in the context of the aforementioned method refers to a comparison of biological activities.

The aforementioned method of the present invention is a screening method. The method of the present invention, preferably, may be carried out in vitro or ex vivo, and, more preferably, in vivo. By applying the aforementioned method compounds can be identified that are capable of influencing the biological activity of the MuRF1 and MuRF2 polypeptide. Preferably, the identified compounds inhibit the biological activity of the of the MuRF1 and MuRF2 polypeptide. Compounds that are identified by said method will be, preferably, promising candidates for the stimulation of de novo muscle protein synthesis and for ROS-protection, and thus, for the prevention or treatment of myocellular damage during stress and chronic heart failure, and for the prevention of muscle wasting conditions during sepsis and diabetes. Finally, stimulation of lipid metabolism will lead to a reduction of body fat and thus, combined MuRF1 and MuRF2 inhibitors will be suitable compounds for the treatment or prevention of adipositas. Thus, the aforementioned method also relates to a method for identifying a compound capable for the treatment and prevention of the aforementioned conditions, disorders and diseases.

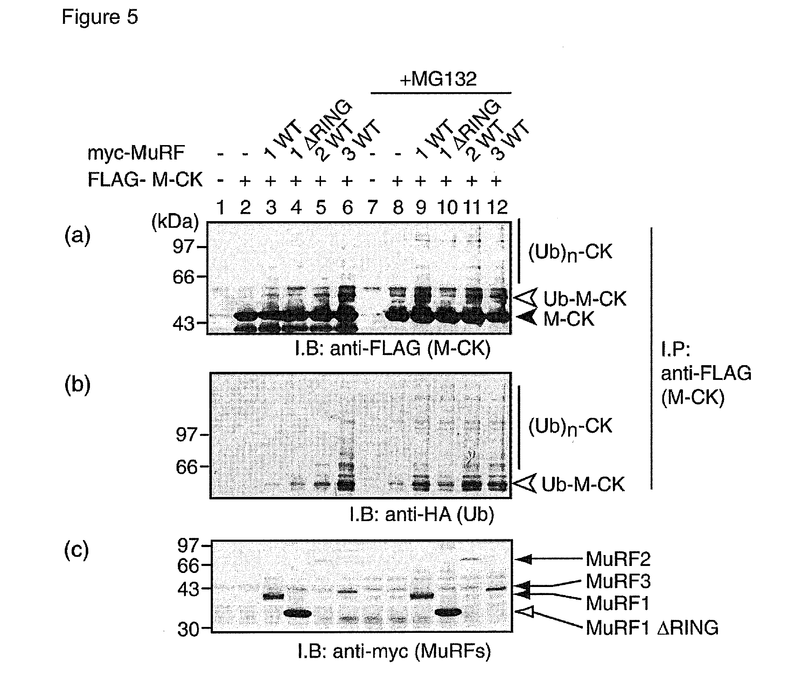

As used herein, the term "biological activity", preferably, refers to the cellular function of the MuRF1 and MuRF2 polypeptides, respectively. It is well known in the art that MuRF1 is an ubiquitin ligase. We could show that MuRF2 similarly ubiquitinates its binding protein muscle creatine kinase (Figure 5). Therefore, said term, preferably, relates the enzymatic activity (frequently also referred to as enzyme activity) of the MuRF1 and the MuRF2 polypeptide. Preferably, said enzymatic activity of the MuRF1 and MuRF2 polypeptide is sumoylation activity, and more preferably, ubiquitination activity.

The term "ubiquitination", preferably, relates to formation of a peptide bond between ubiquitin and the side-chain NH₂ of a lysine residue in a target protein Moreover, the term also includes polyubiquitination, thus, covalently linking a second ubiquitin with a first ubiquitin residue that is covalently linked to a target protein, covalently linking a third ubiquitin to said second protein, and so forth. Thus, the term encompasses mono- and polyubiquitination. Preferably, polyubiquitination marks polypeptides for degradation by the proteasome, whereas monoubiquitination does not target a polypeptide for degradation. It is well known that monoubiquitination may alter the activity of the respective target polypeptide (Stelter et al., 2003: Nature 425, 188-191).

The term "sumoylation" as used herein, preferably, relates to a post-translational modification of a target protein by which a Small Ubiquitin-related Modifier (SUMO) protein is attached to a target protein.

The enzyme activity, and, thus, the ubiquitination and/or sumoylation enzyme activity can be assessed by suitable enzyme assays. How to determine ubiquitination and sumoylation activity is well known in the art (for example: Major et al., 2007: Science 316, 1043-1046). For an in vivo ubiquitination assay determining MuRF1/MuRF2 ubiquitination activities in COS9 cells, see Figure 5).

Preferably, the term "biological activity" also relates to the binding function of the MuRF1 and MuRF2 polypeptide and, thus, to the ability of the MuRF1 and MuRF2 polypeptides to bind a suitable binding partner. Said binding partner, preferably, is an interaction polypeptide of the MuRF1 and MuRF2 polypeptide which are described herein below. It is well known in the art that, e.g., enzyme function may be inhibited when the ability of a said enzyme to bind its binding partner and, thus, to recognize its binding partner is decreased. Binding assays are well known in the art. Preferred binding assays are described elsewhere in this specification.

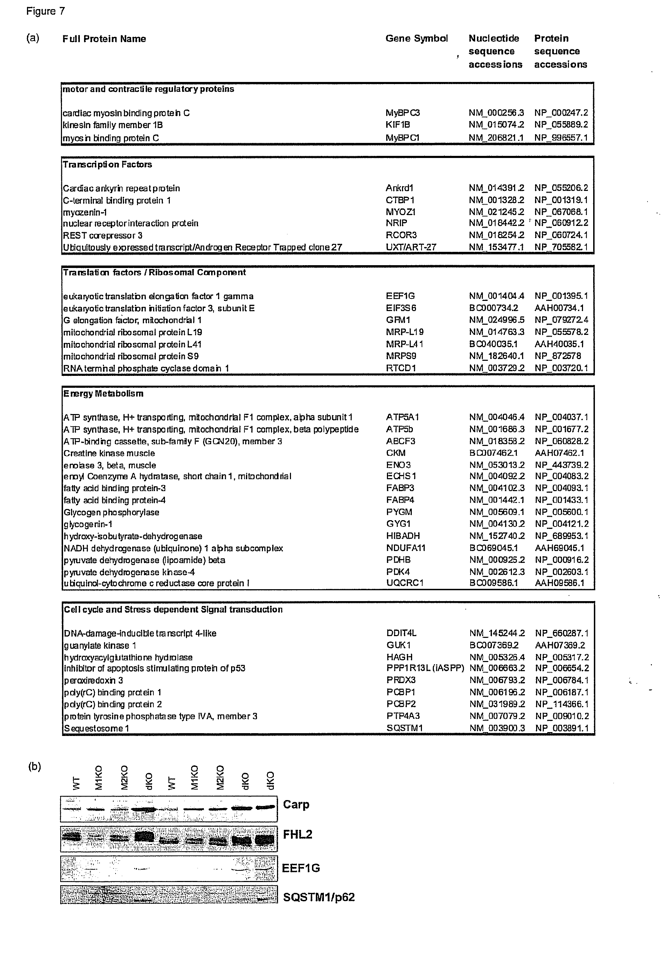

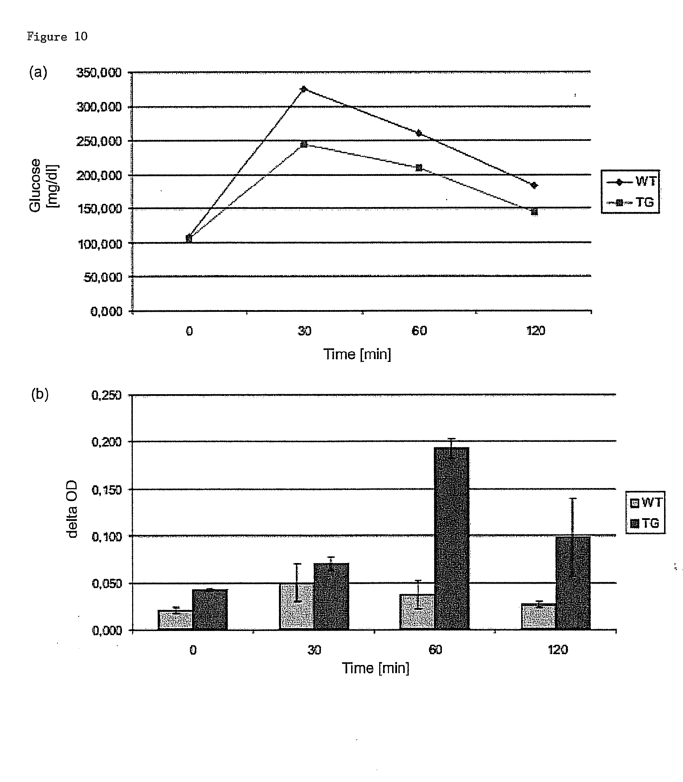

Since the combined inactivation of MuRF1 and MuRF2 has profound and specific effects on the expression of selected binding partners/interaction polypeptides (e.g. expression of CARP, Fhl2, EEF1G become all highly elevated after MuRF1+MuRF2 inactivation, see Figure 7), on the transcription of a specific set of muscle genes (for highly upregulated genes, see Figure 6), on TNF-alpha sensitivity/ROS production (Figure 9), and on insulin secretion (see Figure 10), the biological activity, preferably, may also be determined by determining the interaction polypeptides, or the transcription of said muscles genes, TNF-alpha sensitivity/ROS production or insulin secretion.

Preferably, a reference biological activity is derived from a host cell expressing the MuRF1 polypeptide and the MuRF2 polypeptide to which the tested compound was not administered or to which a lower amount of the tested compound was administered. Preferably, the change of the biological activity is a decrease. Preferably, a decrease of the biological activity of the MuRF1 polypeptide and the MuRF2 polypeptide in the host cell or organism to which the tested compound was administered compared with the reference biological activity indicates that the tested compound is capable of inhibiting the biological activity of the MuRF1 polypeptide and the MuRF2 polypeptide.

Furthermore, the present invention relates to a method for identifying a compound which is capable of influencing the interaction of the MuRF1 and MuRF2 polypeptide and an interaction polypeptide capable of interacting with the MuRF1 and MuRF2 polypeptide, comprising the steps of
a) contacting said MuRF1 polypeptide with said interaction polypeptide and the compound to be tested,
b) contacting said MuRF2 polypeptide with said interaction polypeptide and the compound to be tested,
c) determining the interaction between said MuRF1 polypeptide and said interaction polypeptide,
d) determining the interaction between said MuRF2 polypeptide and said interaction polypeptide,
e) comparing the interaction as determined in step c) with a suitable reference for the MuRF1 polypeptide, and
f) comparing the interaction as determined in step d) with a suitable reference for the MuRF2 polypeptide,
wherein a change of the interaction as determined in step c) and in step d) compared with the corresponding reference indicates that the tested compound influences the interaction between the MuRF1 and MuRF2 polypeptide and said interaction polypeptide.

The aforementioned method, preferably, is an in vitro method.

The term "interaction polypeptide", preferably, relates to a polypeptide that interacts with both the MuRF1 and the MuRF2 polypeptide. The term "interaction", preferably, includes binding, regulation and modification.

The term "binding" relates to a physical association, and, preferably, to a non-covalent physical association between the interaction polypeptide and the MuRF1 polypeptide or between the interaction polypeptide and the MuRF2 polypeptide. Preferably, the physical association is specific.

The term "modification", preferably, relates to, phosphorylation such as phosphorylation of a serine, threonine and/or tyrosine residue, glycosylation, prenylation, myristoylation, farnesylation, attachment of a fatty acid, attachment of a GPI anchor, acetylation, methylation and nucleotidylation such as ADP-ribosylation, and more preferably to ubiquitination and sumoylation. It is contemplated by the aforementioned method that (i) the interaction polypeptide is modified by the MuRF1 and MuRF2 polypeptide and/or (ii) the interaction polypeptide modifies the MuRF1 and MuRF2 polypeptides. Regarding (i) the most preferred modifications are ubiquitination (mono- or polyubiquitination) or sumoylation (see above). It is to be understood that a modification may also involve binding.

It is contemplated that the interaction polypeptide interacts both with the MuRF1 polypeptide and the MuRF2 polypeptide. This interaction, preferably, may be (i) an interaction of the MuRF1 polypeptide, the MuRF2 polypeptide and the interaction polypeptide (leading to a complex of at least three polypeptides comprising MuRF1, MuRF2 and the interaction polypeptide), and, more preferably, (ii) an interaction of the MuRF1 polypeptide or the MuRF2 polypeptide with the interaction polypeptide (thus a complex of two polypeptides). However, is to be understood that the interaction polypeptide, preferably, interacts with both the MuRF1 and MuRF2 polypeptide.

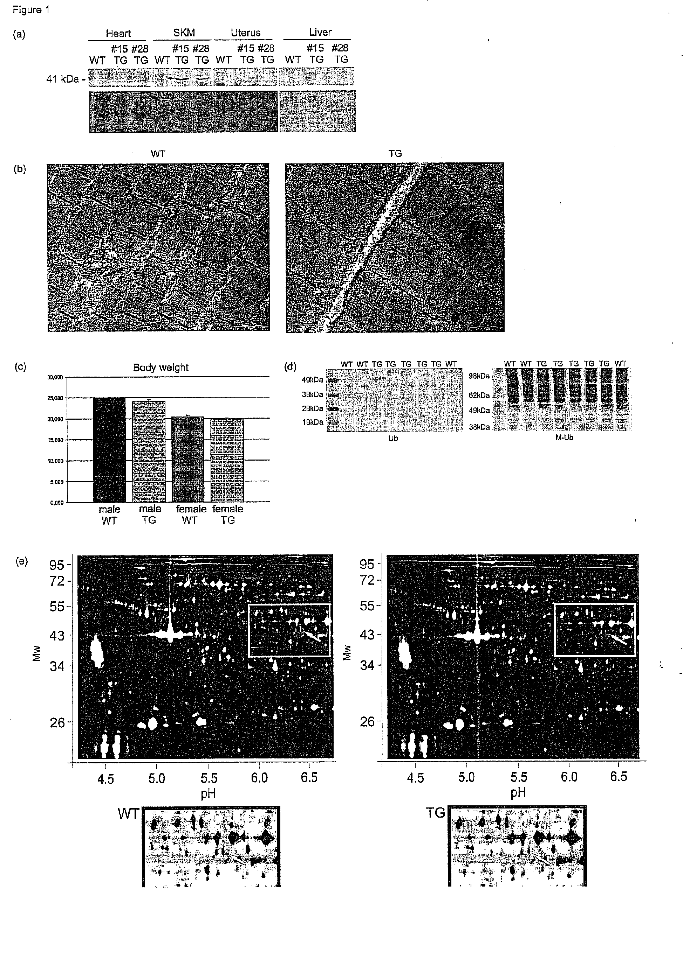

Preferred interaction polypeptides in the context of the present invention are polypeptides having a sequence indicated by the following Genbank accession numbers: NP_060828.2, NP_004037.1, NP_001677.2, NP_055206.2, NP_001319.1, AAH07462.1, NP_660287.1, NP_004083.2, NP_001395.1, AAH00734.1, NP_443739.2, NP_004093.1, NP_001433.1, NP_079272.4, AAH07369.2, NP_005317.2, NP_689953.1, NP_055889.2, AAH40035.1, NP_055578.2, NP_872578.1, NP_996557.1, NP_067068.1, AAH69045.1, NP_060912.2, NP_006187.1, NP_114366.1, NP_006784.1, NP_000916.2, NP_002603.1, NP_006654.2, NP_009010.2, NP_060724.1, NP_003720.1, NP_003891.1, AAH09586.1, NP_705582.1; NP_005600.1; NP_004121.2; NP_000247.2; NP_963851.2 (for the corresponding gene and protein names, see Figure 7). Of these, most preferred interaction polypeptides are EEF1G (NP_001395.1), CARP (NP_055206.2), FHL2 (NP_963851.2), and sequestosome-1 (NP_003891.1; see Figure 7b), PDHB (NP_000916.2; Figure 1), PDK4 (NP_002612.3; see Figure 6), HAGH (NP_005317.2), and PRDX3 (NP_006784.1; see also Figure 9). Their preferred regulation by MuRF1 and MuRF2 is indicated by their dysregulation in the proteome or transcriptome in dKO mice, respectively, or lack of their appropriate antioxidant functioning during metabolic stress. A compound that would inhibit the interaction of the MuRF1 and MuRF2 polypeptide with eEF1G or GFM1 would allow to stabilize protein translation in muscle tissues when being stressed, for example by TNF-alpha during sepsis or chronic heart failure (see Figure Figure 9b). A compound that would inhibit the interaction of the MuRF1 and MuRF2 polypeptide with PDHB and PDK4 would prevent the MuRF1/2-dependent downregulation of glucose metabolism (see Figure Figure 10). A compound that would inhibit the interaction of the MuRF1 and MuRF2 polypeptide with HAGH (synonyms: hydroxyacyl-glutathione hydrolase or glyoxalase II) and PRDX3 (peroxiredoxin-3) would inhibit the production of reactive oxygen species and thus prevent myocellular damage (see Figure 9d).

Thus, the combined MuRF1/MuRF2 inhibitors will improve muscle metabolism and myocellular integrity in particular during the following chronic conditions, disorders, and diseases: Chronic heart failure promoted by increased circulatory cytokine levels such as TNF-alpha, diabetes, causing damage on myocytes by impaired glycolysis and oxidative stress, and sarcopenia during ageing where a primary elevation of muscle protein synthesis is beneficial to maintain sarcomeres in states with elevated catabolism due to chronically inactive lifestyle and/or in activated inflammatory signalling pathways, such as TNF-alpha. Therefore, inhibitors of MuRF1 and MuRF2 will be valuable for treating, preventing and ameliorating, e.g., the aforementioned conditions, disorders and diseases.

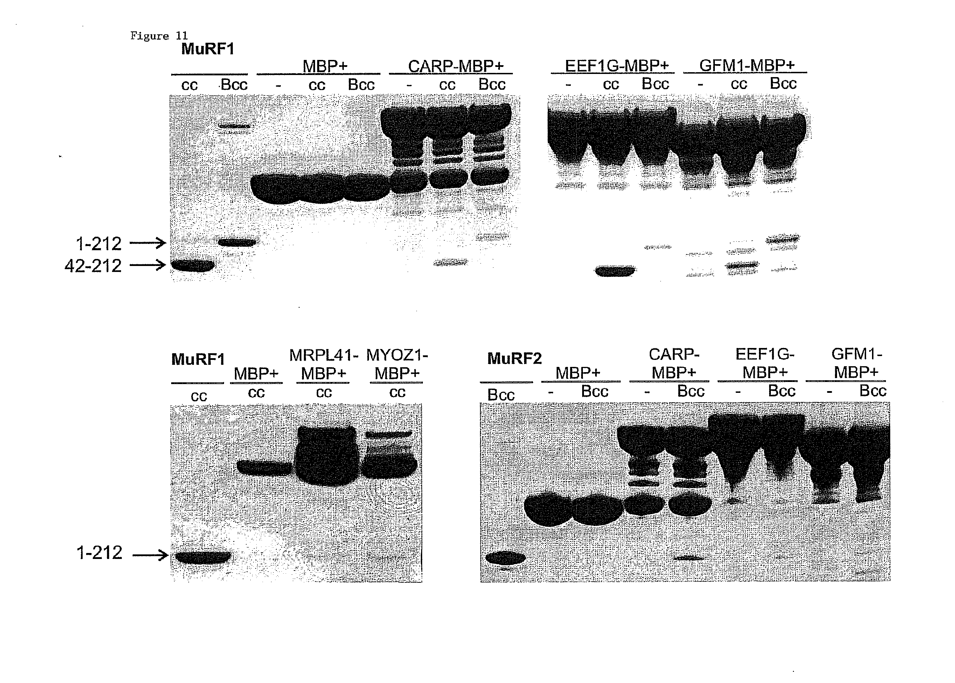

Moreover, the person skilled in the art is capable of determining more interaction polypeptides capable of interacting with the MuRF1 and the MuRF2 polypeptide. These interaction polypeptides can be determined by carrying out well know methods such as yeast two hybrid screens (Figure 7), pulldown assays (see Figure 11), or proteome surveys (see Figure 1e).

Preferably, the MuRF1 and MuRF2 polypeptides are derived from vertebrates, more preferably, from mammals, and even more preferably, from rodents. The most preferred MuRF1 and MuRF2 polypeptides are the human MuRF1 and MuRF2 polypeptides (see AJ291712.1 and AJ291713.1). Moreover, preferred interaction polypeptides are human interaction polypeptides.

It is to be understood that instead of the full size MuRF1 and MuRF2 polypeptides variants or functional domains thereof can be used in the aforementioned method. Moreover, instead of a full size interaction polypeptide variants or functional domains thereof may be used. It is also contemplated that the aforementioned polypeptides or fragments of variants comprise specific labels which facilitate determination of polypeptide/polypeptide interaction.

The term "variant" as used herein, preferably, relates to a polypeptide which differs from the related polypeptide in one or more amino acid residue. Such a variant can be generated by insertion, deletion and/or substitution of one or more amino acids. Preferably, a variant in the context of the present application is functional, thus a variant retains the biological activity of the corresponding polypeptide to a large extend. Preferably, a variant differs from the corresponding polypeptide not more than 30%, not more than 20%, more preferably not more than 10%, and most preferably not more than 5%.

Functional domains of the MuRF1 and MuRF2 polypeptide (and thus domains which are involved in binding the interaction polypeptide and/or modifying the interaction polypeptide or being modified by the interaction polypeptide) are described elsewhere in this specification.

"A compound capable of influencing the interaction of the MuRF1 and MuRF2 polypeptide and an interaction polypeptide", preferably, is a compound that changes the said interaction. Preferably, said compound reduces and, thus, inhibits said interaction. Preferred changes are described elsewhere herein. It is to be understood that said compound influences the interaction between the MuRF1 polypeptide and an interaction polypeptide as well as the interaction between the MuRF2 polypeptide and said interaction polypeptide.

Contacting the MuRF1 and/or the MuRF2 polypeptide with an interaction polypeptide and a compound to be tested can be done by any method deemed appropriate. Preferably, contacting is done by bringing the mentioned components into proximity. This can be achieved, e.g., by simply bringing said components into one solution.

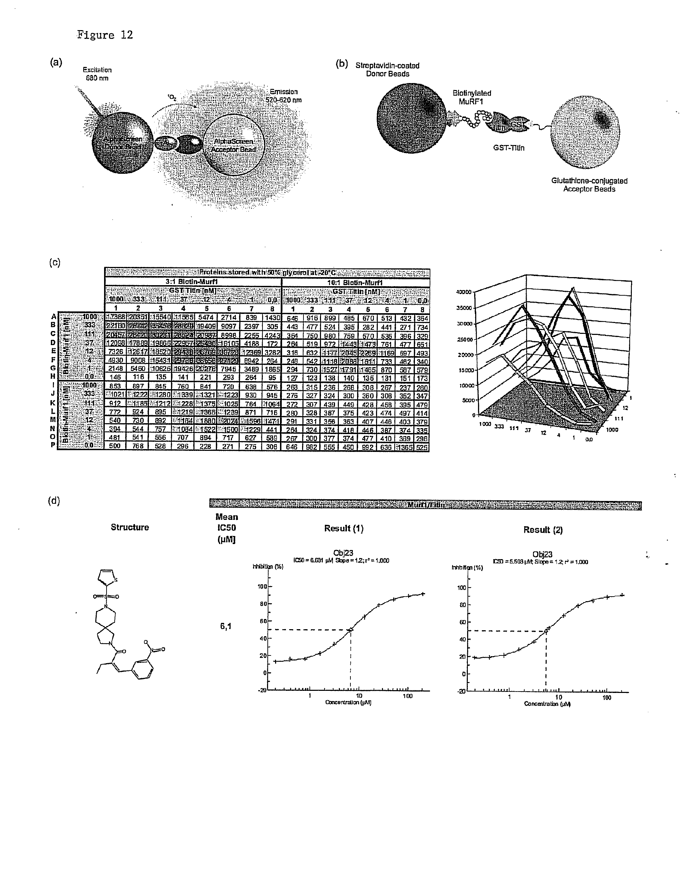

The determination of the interaction between the polypeptides referred to above can be done by any method deemed appropriate. How to determine the interaction may depend on how the polypeptides interact with each other. Preferred methods for determining polypeptide-polypeptide interaction are coimmunoprecipitation assays (also known as pull down assays), fluorescence resonance energy transfer (FRET), and fluorescent polymerization and iso-calorimetric titration. A particularly preferred method for determining the interaction between polypeptides is the amplified luminescent proximity homogenous assay, frequently also referred to as Alpha-Screen (Sehr et al., 2007: J Biomol Screen 12, 560-567; see e.g. Figure 12).

Moreover, if the interaction is based on modification of the interaction polypeptide by the MuRF1 and MuRF2 polypeptide (and vice versa), the interaction, preferably, is determined by suitable enzyme assays which allow measuring the enzymatic activity in a suitable sample comprising an interaction polypeptide, a compound to be tested and the MuRF1 polypeptide and/or the MuRF2 polypeptide (it is to be understood the sample may comprise more components). Since MuRF1 and MuRF2 are ubiquitin ligases, interaction with an interaction protein is, preferably, determined by determining the ubiquitination and/or sumoylation of said interaction polypeptide. E.g., if the interaction polypeptide is ubiquitinated by the MuRF1 polypeptide, then ubiquitination assays are, preferably, carried out in order to determine interaction between said interaction polypeptide and said MuRF1 polypeptide.

Suitable references in the context of the aforementioned method, preferably, can be derived by determining the interaction between the MuRF1 polypeptide and the interaction polypeptide (reference for MuRF1) and determining the interaction between the MuRF2 polypeptide and said interaction polypeptide (reference for MuRF2) without contacting said polypeptides with a compound to be tested. Alternatively, e.g. a lower amount of said compound may be used for the reference.

In the context with the aforementioned method, preferably, the change is a decrease. Thus, a decreased interaction between the MuRF1 polypeptide and an interaction polypeptide and a decreased interaction between the MuRF2 polypeptide and said interaction polypeptide indicates that the tested compound is capable of inhibiting interaction between the interaction polypeptide and (i) the MuRF1 polypeptide and (ii) the MuRF2 polypeptide.

A compound identified by the method of the present invention will be a promising candidate for treating, prevention and ameliorating a condition, disorder or disease referred to herein. Accordingly said compound, particularly, will be a candidate for improving muscle metabolism and myocellular integrity in the following chronic states: Chronic heart failure promoted by increased cytokine levels in the blood, diabetes, causing oxidative stress and damage in myocytes, wasting conditions, and sarcopenia during ageing or chronical inflammatory states. Moreover, said compound will be a candidate for increasing the ratio of body muscle mass to body fat, E.G. decreasing body fat while at the same time increasing muscle mass strengthening myofibrils.

In a preferred embodiment of the aforementioned method the MuRF1 polypeptide and the MuRF2 polypeptide are concertedly, preferably simultaneously contacted with the compound to be tested and an interaction polypeptide.

Thus, a preferred embodiment of the aforementioned method is a method for identifying a compound which is capable of influencing the interaction of the MuRF1 and MuRF2 polypeptide and an interaction polypeptide capable of interacting with the MuRF1 and MuRF2 polypeptide, comprising the steps of
a) contacting said MuRF1 polypeptide and said MuRF2 polypeptide with said interaction polypeptide and the compound to be tested,
b) determining the interaction between said MuRF1 and MuRF2 polypeptide and said interaction polypeptide,
c) comparing the interaction as determined in step b) with a suitable reference
wherein a change, preferably a decrease, of the interaction as determined in step b) compared with the corresponding reference indicates that the tested compound influences the interaction between the MuRF1 and MuRF2 polypeptide and said interaction polypeptide.

In the studies underlying the present invention yeast two hybrid assays were done in order to fmd interaction polypeptides which are capable of interacting both with the MuRF1 and the MuRF2 polypeptide. Surprisingly, 41 interaction polypeptides were found (see Figure 7). The aforementioned method will, if applied, allow high throughput screens for the identification of compounds which are capable of influencing the interaction of both the MuRF1 and MuRF2 polypeptide with an interaction polypeptide. Therefore, compounds will be identified that inhibit the interaction of the MuRF1 and MuRF2 polypeptide with their metabolic targets.

Moreover, the present invention relates to a composition comprising the MuRF1 polypeptide and the MuRF2 polypeptide, preferably, the human MuRF1 and human MuRF2 polypeptide. Preferably, instead of the full-length polypeptides, said composition may comprise variants, domains or fragments thereof. The term variants, domains or fragments thereof are described elsewhere in this specification. A preferred fragment of the MuRF1 polypeptide is a polypeptide having an amino acid sequence as shown in SEQ ID NO:5 and a preferred fragment of the MuRF2 polypeptide is a polypeptide comprising an amino acid sequence as shown in SEQ ID NO:6 (see also Centner et al., 2001: J Mol Biol 306, 717-726: reporting AJ291713.1 and AJ291712.1).

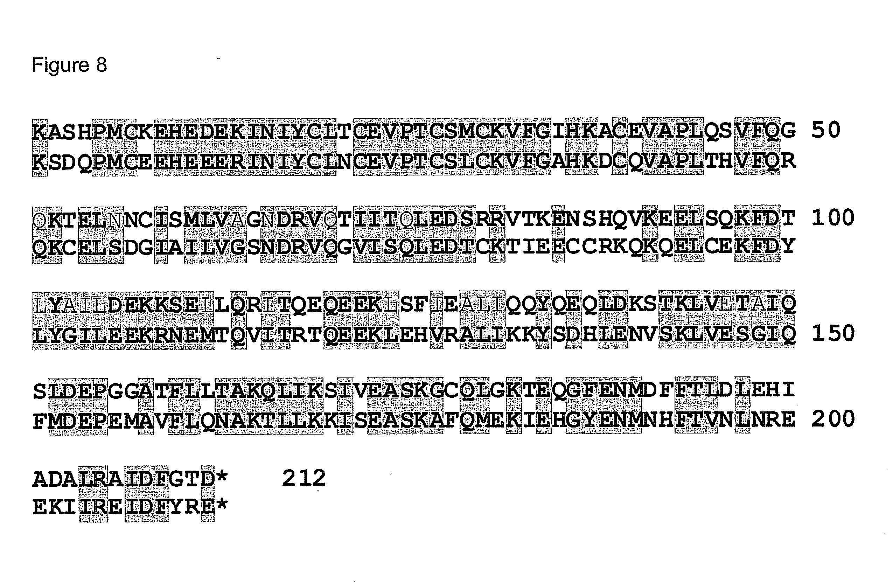

As mentioned above, the combined inactivation of the MuRF1 and the MuRF2 polypeptide had a surprising effect on the de novo synthesis of muscle proteins. The composition comprising the MuRF1 polypeptide and the MuRF2 polypeptide will be very useful for the identification of compounds that will allow treatment or prevention of disorders, diseases and conditions referred to herein, particularly of muscle wasting composition. Moreover, in the studies underlying the present invention fragments of both the human MuRF1 and MuRF2 polypeptide were identified which are particularly useful for screening methods that allow the identification of inhibitors of said interaction. Specifically, a 212 amino acid residue central fragment of both the MuRF1 and the MuRF2 polypeptide (amino acid sequence as shown SEQ ID NO:5 for the MuRF1 fragment; SEQ ID NO:6 for the MuRF2 fragment) was found being responsible for the interaction/recognition of the interaction polypeptides of both MuRF1 and MuRF2 (for a pulldown assay using this fragment, see Figure 11; for an alignment see Figure 8). In the studies underlying the present invention said fragments interacted with all identified interaction polypeptides. Advantageously, in contrast to the full length MuRF1 and MuRF2 polypeptides, said fragments can be easily expressed in E. coli at high levels and purified in a soluble state. Therefore, these fragments and, thus, a composition comprising these fragments will be valuable tools for the screening of compounds that, preferably, regulate, and, more preferably, inhibit and, thus reduce, the interaction of the MuRF1 and MuRF2 polypeptide with an interaction polypeptide.

Moreover, the present invention relates to a pharmaceutical composition comprising siRNAs directed against polynucleotides comprising a nucleic acid sequence as shown in SEQ ID NO:3 and SEQ ID NO:4. Preferably, said composition allows treating ameliorating or preventing a condition, disease or disorder referred to herein. A particularly preferred siRNA in the context of the present invention has a nucleic acid sequence as shown in SEQ ID NO:15 since it will target both MuRF1 and MuRF2. However, also contemplated are compositions comprising a siRNA mixture, wherein said siRNA mixture comprises siRNAs directed against MuRF1 and MuRF2 (thus at least one siRNA directed against MuRF1 and at least one siRNA directed against MuRF2).

As used herein, the term "RNA interference (RNAi)" refers to selective intracellular degradation of RNA used to silence expression of a selected target gene, i.e. the polynucleotides encoding MuRF1 and MuRF2. RNAi is a process of sequence-specific, post-transcriptional gene silencing in organisms initiated by double-stranded RNA (dsRNA) that is homologous in sequence to the gene to be silenced. The RNAi technique involves small interfering RNAs (siRNAs) that are complementary to target RNAs (encoding a gene of interest) and specifically destroy the known mRNA, thereby diminishing or abolishing gene expression. RNAi is generally used to silence expression of a gene of interest by targeting mRNA, however, any type of RNA is encompassed by the RNAi methods of the invention. Briefly, the process of RNAi in the cell is initiated by long double stranded RNAs (dsRNAs) being cleaved by a ribonuclease, thus producing siRNA duplexes. The siRNA binds to another intracellular enzyme complex which is thereby activated to target whatever mRNA molecules are homologous (or complementary) to the siRNA sequence. The function of the complex is to target the homologous mRNA molecule through base pairing interactions between one of the siRNA strands and the target mRNA. The mRNA is then cleaved approximately 12 nucleotides from the 3' terminus of the siRNA and degraded. In this manner, specific mRNAs can be targeted and degraded, thereby resulting in a loss of protein expression from the targeted mRNA. A complementary nucleotide sequence as used herein refers to the region on the RNA strand that is complementary to an RNA transcript of a portion of the target gene. The term "dsRNA" refers to RNA having a duplex structure comprising two complementary and anti-parallel nucleic acid strands. Not all nucleotides of a dsRNA necessarily exhibit complete Watson-Crick base pairs; the two RNA strands may be substantially complementary. The RNA strands forming the dsRNA may have the same or a different number of nucleotides, with the maximum number of base pairs being the number of nucleotides in the shortest strand of the dsRNA. Preferably, the dsRNA is no more than 49, more preferably less than 25, and most preferably between 19 and 23, nucleotides in length. dsRNAs of this length are particularly efficient in inhibiting the expression of the target gene using RNAi techniques. dsRNAs are subsequently degraded by a ribonuclease enzyme into short interfering RNAs (siRNAs).

RNAi is mediated by small interfering RNAs (siRNAs). The term "small interfering RNA" or "siRNA" refers to a nucleic acid molecule which is a double stranded RNA agent that is complementary to i.e., able to base-pair with, a portion of a target RNA (generally mRNA), i.e. the polynucleotide of the present invention being RNA. siRNA acts to specifically guide enzymes in the host cell to cleave the target RNA. By virtue of the specificity of the siRNA sequence and its homology to the RNA target, siRNA is able to cause cleavage of the target RNA strand, thereby inactivating the target RNA molecule. Preferably, the siRNA which is sufficient to mediate RNAi comprises a nucleic acid sequence comprising an inverted repeat fragment of the target gene and the coding region of the gene of interest (or portion thereof).

Also preferably, a nucleic acid sequence encoding a siRNA comprising a sequence sufficiently complementary to a target gene is operatively linked to a expression control sequence. Thus, the mediation of RNAi to inhibit expression of the target gene can be modulated by said expression control sequence. Preferred expression control sequences are those which can be regulated by an exogenous stimulus, such as the tet operator whose activity can be regulated by tetracycline or heat inducible promoters. Alternatively, an expression control sequence may be used which allows tissue- specific expression of the siRNA.

The complementary regions of the siRNA allow sufficient hybridization of the siRNA to the target RNA and thus mediate RNAi. In mammalian cells, siRNAs are approximately 21-25 nucleotides in length (Tuschl et al., 1999: Genes Dev 13, 3191-3197; Elbashir et al., 2001: Nature 411, 494-498). The siRNA sequence needs to be of sufficient length to bring the siRNA and target RNA together through complementary base-pairing interactions. The siRNA used with the Tet expression system of the invention may be of varying lengths. The length of the siRNA is preferably greater than or equal to ten nucleotides and of sufficient length to stably interact with the target RNA; specifically 15-30 nucleotides; more specifically any integer between 15 and 30 nucleotides, most preferably 15, 16, 17, 18, 19, 20, 21, 22, 23, 24, 25, 26, 27, 28, 29, and 30. By "sufficient length" is meant an oligonucleotide of greater than or equal to 15 nucleotides that is of a length great enough to provide the intended function under the expected condition. By "stably interact" is meant interaction of the small interfering RNA with target nucleic acid (e.g., by forming hydrogen bonds with complementary nucleotides in the target under physiological conditions).

Generally, such complementarity is 100% between the siRNA and the RNA target, but can be less if desired, preferably 91%, 92%, 93%, 94%, 95%, 96%, 97%, 98%, or 99%. For example, 19 bases out of 21 bases may be base-paired. In some instances, where selection between various allelic variants is desired, 100% complementary to the target gene is required in order to effectively discern the target sequence from the other allelic sequence. When selecting between allelic targets, choice of length is also an important factor because it is the other factor involved in the percent complementary and the ability to differentiate between allelic differences.

Methods relating to the use of RNAi to silence genes in organisms, including C. elegans, Drosophila, plants, and mammals, are known in the art (see, for example, Fire et al., 1998: Nature 391, 806-811; Fire, 1999: Trends Genet. 15, 358-363; Sharp, 2001: Genes Dev. 15, 485-490; Hammond et al., 2001: Nature Rev. Genet. 2, 1110-1119; Tuschl, 2001: Chem. Biochem. 2, 239-245; Hamilton et al., 1999: Science 286, 950-952; Hammond et al., 2000: Nature 404, 293-296; Zamore et al., 2000: Cell 101, 25-33; Bernstein et al., 2001: Nature 409, 363-366; Elbashir et al., 2001: Genes Dev. 15, 188-200; WO 0129058; WO 09932619; and Elbashir et al., 2001: Nature 411, 494-498).

RNAi may be used to specifically inhibit expression of the polynucleotides of the present invention in vivo. Accordingly, it may be used for therapeutic approaches for diseases or disorders which are accompanied with an altered expression of the polynucleotides of the present invention, e.g., an enhanced expression or a expression of the polynucleotides at wrong locations. For such therapeutic approaches, expression constructs for siRNA may be introduced into target cells of the host which suffer from altered polynucleotide expression. Accordingly, siRNA may be combined efficiently with the available gene therapy approaches.

Moreover, the present invention relates to a method for diagnosing a muscle wasting condition or for assessing the risk of a muscle wasting condition in a subject
a) determining the amount or biological activity of the MuRF1 and MuRF2 polypeptide in a sample of a subject suspected to suffer from a muscle wasting condition or to be at risk of a muscle wasting condition,
b) comparing the amount or the biological activity of the MuRF1 and MuRF2 polypeptide as determined in step a) to a reference amount or reference biological activity, and
c) diagnosing a muscle wasting condition or assessing the risk of a muscle wasting condition based on the, thus, obtained information.

The both aforementioned methods, preferably, are in vitro methods. Moreover, they may comprise steps in addition to those explicitly mentioned above. For example, further steps may relate to sample pre-treatments or evaluation of the results obtained by the method. The method of the present invention may be also used for monitoring, confirmation, and subclassification of a condition referred to herein. The method may be carried out manually or assisted by automation. Preferably, step (a) and/or (b) may in total or in part be assisted by automation, e.g., by a suitable robotic and sensory equipment for the determination in step (a) or a computer-implemented comparison in step (b) and/or (c).

The term "identifying" as used in the context with the method for diagnosis herein means assessing whether a subject will suffer from a muscle wasting condition or not or whether a subject is at risk of a muscle wasting condition or not. As will be understood by those skilled in the art, such an assessment is usually not intended to be correct for all (i.e. 100%) of the subjects to be identified. The term, however, requires that a statistically significant portion of subjects can be identified (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden (Statistics for Research, John Wiley & Sons, New York 1983). Preferred confidence intervals are at least 90%, at least 95%, at least 97%, at least 98% or at least 99 %. The p-values are, preferably, 0.1, 0.05, 0.01, 0.005, or 0.0001. More preferably, at least 60%, at least 70%, at least 80% or at least 90% of the subjects of a population can be properly identified by the method of the present invention.

The term "subject" as used herein relates to animals, preferably mammals, and, more preferably, humans.

"A muscle wasting condition" in the context of the aforementioned method, preferably is a condition in which the synthesis of new muscle proteins is lower than muscle protein degradation. Preferably, said condition is unwanted. Preferred muscle wasting conditions in the context of the present invention are sarcopenia during wasting states, decrease of muscle mass during sepsis, decrease of muscle mass that accompanies diabetes and ageing, and decrease of muscle mass that is caused by long term immobilization.

The term "sample" in the context with a method for diagnosis or a method for assessing the risk of a certain condition refers to a sample of a body fluid, to a sample of separated cells or to a sample from a tissue or an organ. Samples of body fluids can be obtained by well known techniques and include, preferably, samples of blood, plasma, serum, or urine, more preferably, samples of blood, plasma or serum. Tissue or organ samples may be obtained from any tissue or organ by, e.g., biopsy. Separated cells may be obtained from the body fluids or the tissues or organs by separating techniques such as centrifugation or cell sorting. Preferably, cell-, tissue- or organ samples are obtained from those cells, tissues or organs which express or produce the peptides referred to herein.

Accordingly, the term "reference amount" as used in the context with the method of diagnosis refers to an amount which allows assessing whether a subject suffers from a muscle wasting condition or is at risk of suffering from a muscle wasting condition. Thus, a suitable reference amount for diagnosing a muscle wasting condition, preferably, may be derived from a subject who does not suffer from a muscle wasting condition. Accordingly, a reference amount for assessing the risk of a muscle wasting condition, preferably, may be derived from a subject who is not at risk of a muscle wasting condition. It is to be understood that a reference amount for the MuRF1 polypeptide and a reference amount for the MuRF2 polypeptide is required in order to diagnose a muscle wasting condition or the risk thereof.

Particularly, an amount or a biological activity of the MuRF1 polypeptide and an amount or a biological activity of the MuRF2 polypeptide larger, more preferably, 1.5 larger and, most preferably, 2.0 fold larger than the corresponding reference amounts indicate that a subject suffers from a muscle wasting condition, preferably, a metabolic muscle disease condition or is at risk thereof.

Advantageously, it was shown that the significant reduction of the amount of the MuRF1 and the MuRF2 polypeptide significantly stimulated de-novo synthesis of muscle proteins.

Thus, determining the amount of the MuRF1 and MuRF2 polypeptide in a sample of a subject will allow diagnosing a muscle wasting condition or allow assessing the risk thereof. If applied the aforementioned methods for diagnosis will be very beneficial for the health system, since a muscle wasting condition can be reliably diagnosed or a risk thereof of can be reliably assessed. Preferably, a subject who suffers from a muscle wasting condition or is at risk thereof is treated accordingly.

Moreover, the present invention relates to a method for identifying a compound capable for the treatment or prevention of diabetes, metabolic syndrome, insulin resistance or chronic heart failure, comprising the steps of
a) administering a test compound to a host cell or a non-human organism expressing the MuRF1 and/or the MuRF2 polypeptide
b) determining the biological activity of the MuRF1 polypeptide and/or the MuRF2 polypeptide, and
c) comparing the biological activity as determined in step b) with a suitable reference biological activity,
wherein a change, preferably a decrease, of the biological activity of the MuRF1 polypeptide and/or the MuRF2 polypeptide as determined in step b) compared with the reference biological activity indicates that the tested compound is capable of influencing the biological activity of the MuRF1 polypeptide and/or the MuRF2 polypeptide and, thus, is capable for treatment or prevention of diabetes, metabolic syndrome, insulin resistance or chronic heart failure.

Preferably, a thus identified compound also allows the treatment and prevention of myocellular damage caused by diabetes.

Moreover, the present invention relates to a method for identifying a compound capable for the treatment or prevention of diabetes, metabolic syndrome, insulin resistance or chronic heart failure, comprising the steps of
a) administering a test compound to a host cell or a non-human organism expressing the MuRF1 and/or the MuRF2 polypeptide
b) determining the amount of the MuRF1 polypeptide and/or the MuRF2 polypeptide, and
c) comparing the amount as determined in step b) with a suitable reference amount,
wherein a change, preferably a decrease, of the amount of the MuRF1 polypeptide and/or the MuRF2 polypeptide as determined in step b) compared with the reference amount indicates that the tested compound is capable for the treatment or prevention of diabetes, metabolic syndrome, insulin resistance or chronic heart failure.

Preferably, a thus identified compound also allows the treatment and prevention of myocellular damage caused by diabetes.

The aforementioned method is a screening method. It is contemplated by the present invention that the method is can be carried out in vivo or in vitro.

The host cell, preferably, is a vertebrate, more preferably, a mammal and, even, more preferably a rodent and most preferably a rat or a mouse host cell. Accordingly, the non-human organism, preferably, is a vertebrate, more preferably, a mammal and, even, more preferably a rodent and most preferably a rat or a mouse. Preferably, said host cell is a muscle cell, more preferably a striated muscle cell, even more preferably a skeletal muscle cell, and most preferably, a myocardial muscle cell.

A suitable reference amount (or biological activity) for MuRF1 and MuRF2, preferably, can be determined by carrying out the aforementioned method without administering a compound to be tested to said host cell or non-human organism. Accordingly, a suitable reference biological activity can be determined.

A decrease of the amount of the MuRF1 polypeptide and/or the MuRF2 polypeptide in the host cell or organism to which the compound to be tested was administered compared with the reference amount for the MuRF1 polypeptide and/or for the MuRF2 polypeptide, preferably, indicates that said compound is capable for the treatment or prevention of diabetes, metabolic syndrome, insulin resistance or chronic heart failure.

Diabetes according to the present invention relates to all forms of diabetes mellitus, including type 1, type 2 and gestional diabetes. Particularly, diabetes relates to type 1 and type 2 diabetes, most particularly to type 2 diabetes. Definitions of diabetes mellitus are known to the person skilled in the art and diagnostic criteria have been established by the World Health Organization (WHO) in 1985 and 1999, as well as by the American Diabetes Association (ADA) in 1997. Any patient fulfilling the criteria according to one or more of these definitions is considered a diabetes patient. Preferably, the diabetes patient is defined according to the WHO 1999 criteria.

Type 1 diabetes is also known as juvenile diabetes or insulin-dependent diabetes mellitus (IDDM). Type 1 diabetes can be caused immunologically (subtype A) and/or it can be idiopathic (subtype B). Type 2 diabetes is also known as adult-onset diabetes or non-insulin-dependent diabetes mellitus (NIDDM). Type 2 diabetes can either be accompanied by adipositas (type 2a) or not be accompanied by adipositas (type 2b). Further types of diabetes are, e.g., caused by genetic defects, diseases of the exocrine pancreas, endocrinopathies, and influences of chemicals or pharmaceutical drugs.

The term "heart failure" as used herein relates to an impaired systolic and/or diastolic function of the heart. Preferably, heart failure referred to herein is also chronic heart failure. More preferably, heart failure as used herein is accompanied by a left ventricular ejection fraction (LVEF) of less than 60%, of 40% to 60% or of less than 40%.

Advantageously, it was shown in the studies underlying the present invention that mice which overexpress the MuRF1 protein skeletal muscle cells require elevated levels of insulin. Specifically, mice were generated stably expressing the MuRF1 polypeptide under the control of a skeletal muscle specific MCK-promoter, that has been optimized by site-directed mutagenesis to restrict expression to skeletal-muscle tissues (Grill et al., 2003: Transgenic Res 12, 33-43). The effects on skeletal muscle were discrete and limited to a dysregulation of pyruvate dehydrogenase (PDH) and other enzymes in charge of glycolytic and mitochondrial oxidative phosphorylation. Interestingly, changes in glycogen liver content and insulin secretion were observed, indicating that although MuRF1 expression was skeletal-muscle specific, elevated muscle MuRF1 levels caused also a profound dysregulation of systemic carbohydrate metabolism. In particular, it was found mice that express MuRF1 require for their metabolism permanently elevated insulin levels (see Figure 8). Therefore, inhibition of MuRF1 (by decreasing the amount or biological activity of MuRF1, or by inhibiting interaction with a suitable interaction polypeptide) will also allow a lower requirement of insulin and, thus, provide a promising strategy for the treatment of diabetes and, preferably, also of diabetes related disorders such as insulin resistance and metabolic syndrome. Moreover, it will be a promising candidate for the treatment of chronic heart failure. Additionally, MuRF1 inhibition will at the same time also prevent myocellular damage caused by reactive oxygen species (Figure 9).

Moreover, the present invention relates to method for identifying a compound capable for the treatment or prevention of diabetes and of myocellular damage caused by it, metabolic syndrome, insulin resistance or chronic heart failure comprising the steps of
a) contacting a MuRF1 polypeptide and/or a MuRF2 polypeptide with an interaction polypeptide capable of interacting with the MuRF1 and/or MuRF2 polypeptide, and the compound to be tested,
b) determining the interaction between said interaction polypeptide and (i) the MuRF1 polypeptide and/or (ii) the MuRF2 polypeptide and,
c) comparing the interaction as determined in step b) with a suitable reference (suitable references), and
wherein a change, preferably, a decrease (compared with the reference) of the interaction between the interaction polypeptide and the (i) MuRF1 polypeptide and/or (ii) the MuRF2 polypeptide indicates that the tested compound is capable for the treatment or prevention of diabetes and of myocellular damage caused by it, metabolic syndrome, insulin resistance or chronic heart failure.

Preferably, the interaction polypeptide in the context with the aforementioned method is capable of interacting with either MuRF1 or MuRF2 or both MuRF1 and MuRF2. Preferably, said interaction polypeptide is selected from the group consisting of PDHB (GenBank Accession No: NP_000916.2), PDK4 (NP_002603.1), ENO3 (NP_443739.2), FABP3 (NP_004093.1), FABP4 (NP_001433.1), EEF1G (NP_001395.1), GFM1 (NP_079272.4), HAGH (NP_005317.2), and PRDX3 (NP_006784.1). Preferred interaction polypeptides are indicated by their dysregulated expression in dKO mice or MuRF1 transgenic mice (i.e. mice that overexpress MuRF1 in a skeletal muscle specific manner), (see Figures 1 and 9), by their downregulation under TNF-alpha induced stress (Figure 9), their contribution as protective antioxidants (see Figure Figure 10d), and by their connection to lipid metabolism (FABP3 and FABP4). Other preferred interaction polypeptides are referred to herein elsewhere.

Moreover the present invention relates to a method for diagnosing diabetes, metabolic syndrome, insulin resistance or chronic heart failure or for assessing the risk thereof in a subject
a) determining the amount or biological activity of the MuRF1 and/or MuRF2 polypeptide in a sample of a subject suspected to suffer from diabetes, metabolic syndrome, insulin resistance or chronic heart failure or to be at risk thereof,
b) comparing the amount or the biological activity of the MuRF1 and/or MuRF2 polypeptide as determined in step a) to a reference amount or reference biological activity, and
c) diagnosing diabetes, metabolic syndrome, insulin resistance or chronic heart failure disease or assessing the risk thereof based on the, thus, obtained information.

Moreover, the present invention relates to a host cell specifically overexpressing MuRF1. Preferably, said host cell is a eukaryotic host cell, more preferably to a vertebrate host cell and, even more preferably, to a mammalian host cell, and most preferably a rodent host cell. Rodent host cells, preferably, include mouse cells, rat cells and hamster cells. Preferably, the host cell is a non-human cell. Preferably, said host cell is a muscle cell, more preferably a striated muscle cell, even more preferably a myocardial muscle cell and, most preferably, a skeletal muscle cell. Preferably, a skeletal muscle specific overexpression is achieved by operably linking a polynucleotide to a MCK-promoter (muscle creatinine kinase) promoter, preferably, to a MCK-promoter that has been optimized by site-directed mutagenesis to restrict expression to skeletal-muscle tissues (Grill et al., 2003: Transgenic Res 12, 33-43).

Finally, the present invention relates to the use of a host cell deficient for the MuRF1 and MuRF2 polypeptide or an organism comprising said host cell for the identification of a compound that is suitable for influencing the expression and/or biological activity of MuRF1, MuRF2 or more preferably, both MuRF1 and MuRF2. A, thus, identified compound will be a promising candidate for treating, preventing or ameliorating conditions, disorders or diseases as referred to herein.

All references cited in this specification are herewith incorporated by reference with respect to their entire disclosure content and the disclosure content specifically mentioned in this specification.

The Figures show:
Figure 1. Generation of mouse lines expressing MuRF1 in skeletal muscle tissues.
   (a) Analysis of the tissue expression profiles in the lines under study by Western blots analysis. In the two independent transgenic lines (TG; lines #15 and #28), expression of full length MuRF1 (38 kDa) was detected in quadriceps skeletal muscle (SKM; arrow), but not in wildtype (WT) control extracts. No expression was detected in heart (left ventricle), liver and uterus smooth muscle tissues. (b) Electron microscopy of myofibrils MuRF1-TG and WT mice. MuRF1-TG mice have normal sarcomeric ultrastructure and normal mitochondria.
   (c) MuRF1-TG mice were analyzed at 10 weeks of age. Left: Mice from strain#15, strain #28, and of WT mice did not differ in whole body weight. Also quadriceps skeletal muscles dissected from those mice did not differ in weight (data not shown).
   (d) Total muscle proteins extracted from quadrizeps skeletal muscle were analyzed in Western blots with antibodies specific for ubiquitin (Ub), and multi-ubiquitin (M-Ub). No difference between WT and TG mice were detected.
   (e) The quadrizeps skeletal muscle proteom present in WT and TG mice was analyzed on 2D gels. No global changes in structural muscle proteins are present, inconsistent with the view that MuRF1 induced the degradation of major structural muscle proteins such as myosins or troponin-I. However, pyruvate dehydrogenase was identified by mass-spectrometry as being lowered in TG mice (see inset).
   Thus, expression of MuRF1 in skeletal muscle is not inducing muscle atrophy, or elevated levels of multi-ubiquitin, but affecting metabolic enzymes.
Figure 2. Inactivation of MuRF1/2 genes in the murine genome and creation of a MuRF1+2 double deficient mouse (dKO) model.
   (a) MuRF1 and MuRF2 genes were inactivated by a homologous recombination and by introducing stop codons within their exons 2 and exon 5, respectively. Gene targeting and provision of stop codons in all three frames by the Neo cassette insertion was performed essentially as described in Witt et al., 2006: EMBO J 25, 3843-3855). Genotyping with the primer pairs indicated in (B) indicates inactivation of the MuRF1 or MuRF2 genes.
   (B) RT-PCR detects loss of MuRF1 and MuRF2 transcription in KO mice. MuRF3 transcription is not affected in dKO mice, showing that the phenotype observed in dKO mice develops in the presence of normal MuRF3 levels. Aldolase: Control for input mRNA/cDNA quantity and quality. Primer sequences for RT-PCR detection of MuRF1 and MuRF2 and for controls were: AGAAGTCGGGGGTCAGGGGACG (SEQ ID NO:7, MuRF1 forw); GGTCCATGATCACTTCATGGCGGCACGAGG (SEQ ID NO:8, MuRF1 rev);
      GAGCACTTCTCTGAATTACAAGTCTTTCTCC (SEQ ID NO:9 ,MuRF2 forw);
      GCTGAGAGCCAATGGGCACCACCTCCCC (SEQ ID NO:10 MuRF2 rev);
      GCCCCATCTGCCTGGAGATGTTCTCCAAGCC (SEQ ID NO:11 MuRF3 forw);
      GCCGCTCCTCCTCCAGCCCCGCATTGCCC (SEQ ID NO:12 MURF3 rev);
      AGCTGTCTGACATCGCTCACCG (SEQ ID NO:13 ALDOLASE FORW);
      CACATACTGGCAGCGCTTCAAG (SEQ ID NO:14 ALDOLASE REV).
Figure 3. Inactivation of MuRF1 and MuRF2 stimulates muscle protein synthesis.
   (a) De novo total muscle protein synthesis was determined by measuring incorporation of deuterium-labeled phenylalanine (D5-F) into muscle proteins after injection of D5-F marker. Fractional synthesis rate (% per 48 hours) of total cardiac muscle proteins was determined by injecting D5-phenylalanine i.p. into mice of each genotype (n=6). Total cardiac muscle protein synthesis is elevated in dKO myocardium (p = 0.05 in dKO vs WT, mice aged between 6 and 16 months). Inactivation of MuRF1 and MuRF2 alone had no significant effects on muscle protein synthesis, when compared to WT.
   (b) The levels of multi-ubiquitinated cardiac muscle proteins in single MuRF1-KO (M1KO), MuRF2-KO (M2KO) are not altered, whereas in MuRF1+2 (dKO) myocardium, levels are slightly elevated. This demonstrates that reduced ubiquitination is not the cause of increased heart muscle mass.
   (c) During 2-day dietary amino acid deprivation, de novo skeletal muscle protein synthesis remains protected in MuRF1 KO quadrizeps skeletal muscles, whereas WT skeletal muscle protein synthesis is lowered (methods as in (a)).
Figure 4. Cooperative effects of murine MuRF1 and MuRF2 inactivation on muscle and fat body contents.
   (a) Left: Effect of MuRF1/2-genotypes on heart (ventricles, left) and quadriceps skeletal muscle (right) to body weight ratios. dKO mice maintain cardiac hypertrophy during ageing (dKO: 84 % increase, p=0,001, MuRF1: 24,5 % increase, MuRF2: 19 %). In addition, dKO mice have 38.1 % increased quadriceps to body weight ratios (p=0.001), while MuRF1 KO and MuRF2 KO genotypes have moderate effects on skeletal muscle mass (MuRF1: 16 % increase, p=0.05; MuRF2: 11 % increase, p=0.08). Despite increasing body mass in WT during months 4-18, skeletal muscles remain more hypertrophic in dKO mice during ageing (4-18 month of age, dKO n=27; MuRF1 KO n=52; MuRF2 KO n=81; WT n=49). Right: Hematoxylin/eosin sections indicated that skeletal myofibers from dKO muscle show hypertrophic fibers with slightly augmented cross section areas alternating with normal appearing fibers (mice aged 4 month). Size bar: 100 µm.
   (b) Weight gain of WT and dKO mice during ageing. Left: Between months 4 to 18, dKO mice gain less weight (red; p<0.001) than WT. Right: Dissections revealed that aged dKO mice were leaner (mice aged 18 months).
Figure 5. Ubiquitination assays for MuRFs in COS9 cells. MuRF1, a deletion mutant of MuRF1 missing its RING finger (□RING), MuRF2, or MuRF2 constructs were expressed in COS7 cells; ubiquitination of muscle creatine kinase (M-CK) was tested by immunoblots: (a-c) HA-tagged ubiquitin was co-expressed in COS7 cells with FLAG-tagged M-CK (lanes 2-6 and 8-12) and myc-tagged MuRF1, MuRF1 □RING, MuRF2, or MuRF3 (lanes 3 and 9, 4 and 10, 5 and 11, or 6 and 12, respectively) or mock vector (lanes 2 and 8), or with mock vector alone (lanes 1 and 7). Cells were separated into two groups: after 48 hr of transfection, one group was treated with 25 mM MG132 for 2.5 hr (lanes 1-6), but the other was not (lanes 7-12). The cells were then harvested and lysed. Lysates were analyzed by western blots using an anti-myc antibody (c) or immunoprecipitated with an anti-FLAG antibody under denaturing conditions (a,b). Precipitated proteins were subjected to Western blot analysis using anti-FLAG (a) or anti-HA antibodies (b). Open and closed arrowheads, open and closed arrows indicate Ub-M-CK and M-CK, MuRF1-3 and MuRF1 □RING, respectively. Both MuRF1 and MuRF2 ubiquitinated creatine-kinase in a MG132, and thus in proteasome-dependent manner. Ubiquitination of M-CK by MuRF3 was not stimulated by MG132, indicating that the biological regulation of MuRF3 ubiquitination is distinct from MuRF1 and MuRF2.
Figure 6. Identification of shared MuRF1/MuRF2 target genes by array profiling.
   (a) Insertion of the neomycin cassette introduced stop codons in the MuRF1 or MuRF2 genes at the indicated positions and produced MuRF1-KO (M1KO) and MuRF2-KO (M2KO) mice. Obtained mice were healthy, had normal muscle weights, and transcriptional profiling of their heart and skeletal muscles did not detect significant differences at basal conditions.
   (b) The inactivated MuRF1 and MuRF2 alleles were introduced into the same mouse by breeding. In contrast to single M1KO and M2KO mice, dKO mouse muscle tissues specifically upregulated genes in charge of metabolic stress signaling pathways, Z-disk associated stretch-regulated proteins. Note that DDIT4L, CARP, and PDK4 are also directly interacting with MuRF1 and MuRF2 (see Figure 7).
Figure 7. Shared targets of MuRF1 and MuRF2.
   (a) Shared interactions of MuRF1 and MuRF2 with a set of 41 proteins. Yeast two-hybrid screens of human cardiac and skeletal muscle cDNA libraries using full length MuRF1 and MuRF2 baits identified a set of 41 proteins that have important roles in mitochondrial energy metabolism, contractility, protein synthesis, regulation of cellular stress, and gene transcription. The shown set of 41 proteins all interacts with both MuRF1 and MuRF2. In addition to the gene names, the nucleotide and protein accession numbers in the gene bank and EBI data libraries are listed. Left: full protein name and gene Symbol; center and left: Nucleotide and protein sequence accessions in the public EBI and NCBI data libraries, respectively.
   (b) Western blot studies with specific antibodies tested the expression of a subset from the above 41 genes in single MuRF1, single MuRF2, and in MuRF1+MuRF2 dKO mouse myocardial extracts. A single MuRF1 or MuRF2 allele was sufficient to maintain a normal expression pattern. In contrast, deletion of all four MuRF1 and MuRF2 alleles resulted in a strong upregulation of a set of polypeptides that interact with both MuRF1 and MuRF2 (see Figure 7a). Thus, preferred MuRF1 and MuRF2 targets under basal conditions are CARP, FHL2, EEF1G and SQSTM1.
Figure 8. Sequence comparison of the 212-residue fragments of human MuRF1 (top row) and human MuRF2 (below) that can be expressed soluble in E. coli in high amounts and that can be used for pulldown assays to monitor the interaction with EEF1G and GFM1 (see Figure 11).
Figure 9.
   Protective effects of MuRF1 and MuRF2 against cellular damage caused by TNF-alpha or streptozocin (STZ), two substances, inducing reactive oxygen species.
   (a) Four mice were injected with 100 ng TNF-alpha (TNF). Compared to control mice with mock injections (Co), this lowered EEG1G to GAPDH (used as a control gene) ratios more than twofold (right).
   (b) EEF1G to GAPDH ratios were determined similarly in MuRF1-KO and dKO mice with and without prior TNF-alpha injections. Inactivation of the MuRF1 gene partially protected the translation initiation factor EEF1G; inactivation of both MuRF1 and MuRF2 caused elevated levels of EEF1G; these were also protected from TNF-alpha induced degradation.
   (c) Lethal doses of STZ injected into WT mice caused considerably less lethality in M1KO mice, similar as the anti-oxidant nicotinamide adenine dinucleotide lowers toxicity (Wick et al., 1977: Cancer Res 37, 3901-3903).
   (d) Reactive oxygen species (ROS) in the form of H2O2 were determined 24 hours after TNF-alpha injections in wildtype, M1KO and dKO mice. Extracts were prepared from quadrizeps skeletal muscles, and total H2O2 content was determined with a commercially available kit (Sigma-Aldrich, PD1-1KT). Consistent with the art, TNF-alpha induced about 2.5x fold activation of ROS. In contrast, skeletal muscles of M1KO were partially protected, and dKO mice had lowered levels of reactive oxygen species after TNF-alpha treatment.
Figure 10. MuRF1 expression increases metabolic insulin requirements.
   (a) Male WT and MuRF1-TG mice were starved for 16 hours (n = 8 each) followed by intraperitoneal injection of glucose solution (GTT). Basal glucose was about 100 mg/dl in both WT and in MuRF1-TG mice. Glucose tolerance was enhanced in MuRF1-TG mice as indicated by reduced peak glucose serum concentrations.
   (b) Insulin levels before and after glucose injection were monitored in parallel in the blood. Insulin secretion was hyperactivated in MuRF1-TG mice, causing about 8-fold elevations 60 min after glucose injection. Insulin secretion was also elevated before GTT and remained significantly elevated two hours after glucose injection.
Figure 11.
   Expression of cMuRF1 and cMuRF2 fragments in E. coli and pulldown assays with preferred target sequences. The interaction of selected proteins identified by yeast two-hydrid studies was studied in vitro by pulldowns using expressed MuRF1 Bcc/MuRF2 Bcc (B-Box + coiled-coil domain) and MuRF1cc (coiled -coil domain) constructs. MuRF1cc and MuRF1Bcc (arrows) co-eluted together with CARP, EEF1G, GFM1 Maltose Binding Protein (MBP)-fusion proteins. Below: Left: MuRF1cc co-eluted with myozenin-1/calsarcin-2, and MRP-L41/Pig3 MBP-fusion proteins. Right: MuRF2Bcc co-eluted together with CARP, EEFG1, GFM1 MBP-fusion proteins. Controls: MBP plus MuRF1cc, Bcc, MuRF2Bcc respectively or fusion proteins only. Numbers above the arrows refer to the residues from Figure 8 included in the MuRF1/MuRF2 constructs.
Figure 12. Identification of MuRF1/2 ligand interaction inhibitors.
   (a) Principle of the Alpha screen methodology (for details, see Sehr et al., 2007: J Biomol Screen 12, 560-567).
   (b) Adaptation of the Alpha screen assay to using cMuRF1 and titin for high-throughput screens in search of compounds that inhibit MuRF1 ligand interactions. Expressed 212-residue "MuRF1 Bcc" was labeled with biotin. The MuRF1/MuRF2 binding protein titin (fragment A168-170; see Mrosek M et al., 2007: FASEB J 21, 1383-1392) was expressed as a GST-fusion protein (to allow glutathione-coated beads to interact with titin A168-170). Formation of the MuRF1cc/titin protein complex brings the biotin label and the GST-fusion protein into close physical proximity. This specific complex can detected by the addition of streptavidin- coated donor and glutathione-coated acceptor beads: Donor beads contain a photosensitizer which converts ambient oxygen to, singlet oxygen upon illumination at 680 nm. When being in close physical proximity (about 50 nm), energy is transferred from the singlet oxygen to the acceptor, resulting in photons emitted at 520 - 620 nm.
   (c) Titration series using different ratios of biotinylated MuKF1cc and titin-A168-170 GST-fusion proteins in microtitre wells. Highest signals were obtained with 4 nM biotin-labeled MuRF1 and 12 nM titin-GST fusion protein. Since these reactions were performed in microtitre plates in 10 µl volumes, this chemistry allows to screen 100.00-200.000 compounds economically.
   (d) Example of a compound that inhibits MuRF1/titin interaction at 6.1 µM as identified from a 3000-compound pilot screen described above.
      ...

### Examples

List of gene products shown to be differentially expressed in MuRF1/MuRF2 double knock mouse mice (see also Fig. 6)

**Skeletal muscle**

| **UniGene ID Upregulation** | **Gene Title** | **Gene Symbol** |
|---|---|---|
| NM_020581.1 | angiopoietin-like 4 | Angptl4 |
| NM_133971.1 | ankyrin repeat protein domain 10 | Ankrd10 |
| NM_009694.3 | apolipoprotein B editing complex 2 | Apobec2 |
| NM_001110140.2 | ATPase, Ca++ transporting, cardiac muscle, slow twitch 2 | Atp2a2 |
| NM_009721.5 | ATPase, Na+/K+transporting, beta 1 polypeptide | Atplb1 |
| NM_025505.3 | basic leucine zipper nuclear factor 1 | Blzf1 |
| NM_007621.1 | carbonyl reductase 2 | Cbr2 |
| NM_025680.3 | catenin, beta like | Ctnnbl1 |
| NM_009139.3 | chemokine ligand 6 | Ccl6 |
| NM_013492.1 | clusterin | Clu |
| NM_007705.2 | cold inducible RNA binding protein | Cirbp |
| NM_007572.2 | complement component 1, q subcomponent, alpha polypeptide | Clqa |
| NM_009777.2 | complement component 1, q subcomponent, beta polypeptide | Clqb |
| NM_023143.2 | complement component 1, r subcomponent | C1 r |
| NM_028288.5 | cullin 4B | Cul4b |
| NM_007669.4 | cyclin-dependent kinase inhibitor 1A (P21) | Cdkn1a |
| NM_009994.1 | cytochrome P450, family 1, subfamily b, polypeptide 1 | Cyp1b1 |
| NM_021422.3 | DnaJ (Hsp40) homolog, subfamily A, member 4 | Dnaja4 |
| NM_008748.2 | dual specificity phosphatase 8 | Dusp8 |
| NM_010186.5 | Fc receptor, IgG, high affinity I | Fcgr1 |
| NM_010240.2 | ferritin light chain 1 | Ftl1 |
| NM_001077361.1 | four and a half lim domain-1 | Fhl1 |
| NM_010178.2 | FUS interacting protein (serine-arginine rich) 1 | Fusip1 |
| NM_020590.4 | gamma-aminobutyric acid (GABA(A)) receptor-associated protein-like 1 | Gabarapl1 |
| NM_008129.3 | Glutamate-cysteine ligase | Gclm |
| AK171148.1 | glycogen syrithase-3 (brain) | Gys3 |
| NM_007836.1 | growth arrest and DNA-damage-inducible 45 alpha | Gadd45a |
| NM_008655.1 | growth arrest and DNA-damage-inducible 45 beta | Gadd45b |
| X59728.1 | growth arrest specific 5 | Gas5 |
| NM_207225.1 | histone deacetylase 4 | Hdac4 |
| NM_010500.2 | immediate early response factor 5 | ler5 |
| NM_013563.3 | interleukin 2 receptor, gamma chain | Il2rg |
| NM_013692.2 | Kruppel-like factor 10 | Klf10 |
| NM_027398.2 | Kv channel-interacting protein 1 | Kcnip1 |
| NM_011918.4 | LIM domain binding 3 | Ldb3 |
| NM_011838.4 | Ly6/neurotoxin 1 | Lynx1 |
| NM_010728.2 | Lysyl-oxidase | Lox |
| NM_027209.3 | membrane-spanning 4-domains, subfamily A, member 6B | Ms4a6b |
| NM_011951.2 | mitogen activated protein kinase 14 | Mapk14 |
| NM_011943.2 | mitogen activated protein kinase kinase 6 | Map2k6 |
| NM_201519.2 | mitogen-activated protein kinase kinase kinase kinase 5 | Map4k5 |
| NM_008657.2 | Myogenic factor 6 | Myf6 |
| NM_021503.1 | Myozenin2/calsarcin-1 | Myoz2 |
| NM_008760.4 | osteoglycin | Ogn |
| NM_023324.2 | pellino-1 | Peli1 |
| NM_133249.2 | peroxisome proliferative activated receptor, gamma, coactivator 1 beta | Ppargc1 b |
| NM_008421.2 | potassium voltage gated channel | Kcnc1 |
| AK136952.1 | predicted: serine-type endopeptidase activity | MGI:1928849 |
| NM_022881.4 | regulator of G-protein signaling 18 | Rgs18 |
| NM_026446.3 | regulator of G-protein signaling 19 | Rgs19 |
| NM_011281.1 | Retinoid-related orphan receptor gamma | Rorc |
| NM_153587.2 | ribosomal protein S6 kinase, polypeptide 5 | Rps6ka5 |
| NM_025429.2 | Serpin-like antichymotrypsin | Serpinb1a |
| NM_009252.2 | Serpin-like antichymotrypsin | Serpina3n |
| NM_009254.2 | Serpin-like antichymotrypsin | Serpinb6a |
| NM_027868.2 | solute carrier family 41, member 3 | Slc41a3 |
| NM_025706.2 | TBC1 domain family, member 1 | Tbc1d15 |
| NM_001039392.1 | thymosin, beta 10 | Tmsb10 |
| NM_172703.2 | translation initiation factor 4 gamma 3 | Eif4g3 |
| NM_201256.3 | translation initiation factor 4E binding protein 3 | Eif4ebp3 |
| NM_009418.2 | tripeptidyl peptidase II | Tpp2 |
| NM_011185.3 | uncharacterized ubiquitin ligase | Psmb1 |
| NM_013703.1 | Very low density lipoprotein receptor | Vldlr |

**heart**

| **UniGene ID Upregulation** | **Gene Title** | **Gene Symbol** |
|---|---|---|
| - | similar to myosin, heavy polypeptide 4, skeletal muscle | LOC671894 |
| Mm.214950 | actin, alpha 1, skeletal muscle | Acta1 |
| Mm.19961 | natriuretic peptide precursor type A / ANP type A | Nppa |
| Mm.46514 | myosin, light polypeptide 7, regulatory / MLC | Myl7 |
| Mm.21697 | DNA-damage-inducible transcript 4 | Ddit4 |
| Mm.35789 | synaptopodin 2-like | Synpo2l |
| Mm.181 0 | connective tissue growth factor | Ctgf |
| Mm.390355 | myosin, light polypeptide 4 / MLC | Myl4 |
| Mm.8655 | complement component factor h | Cfh |
| Mm.2740 | natriuretic peptide precursor type B / ANP type B | Nppb |
| Mm.35789 | synaptopodin 2-like | Synpo2l |
| Mm.148913 | actin-binding Rho activating protein | Abra |
| Mm.29132 | sarcolipin | Sln |
| Mm.8655 | complement component factor h | Cfh |
| Mm.389224 | enabled homolog (Drosophila) | Enah |
| Mm.422752 | carboxypeptidase X 2 (M14 family) | Cpxm2 |
| Mm.10279 | CARP | Ankrd1 |
| Mm.223744 | kinesin family member 5B | Kif5b |
| Mm.235547 | pyruvate dehydrogenase kinase, isoenzyme 4 | Pdk4 |
| Mm.200916 | glutathione peroxidase 3 | Gpx3 |
| Mm.37666 | chloride intracellular channel 5 | Clic5 |
| Mm.81562 | integrin beta 1 binding protein 3 | ltgb1bp3 |
| Mm.241109 | restin | Rsn |
| Mm.260456 | vesicle-associated membrane protein, associated protein B and C | Vapb |
| Mm.332941 | leiomodin 2 (cardiac) | Lmod2 |
| Mm.223744 | kinesin family member 5B | Kif5b |
| Mm.333406 | cyclin D2 | Ccnd2 |
| Mm.386769 | nebulin-related anchoring protein | Nrap |
| Mm.28518 | tumor necrosis factor receptor superfamily, member 12a | Tnfrsf12a |
| Mm.36769 | sarcolemma associated protein | Slmap |
| Mm.16974 | ubiquitin specific peptidase 47 | Usp47 |
| Mm.143804 | myotilin | Myot |
| Mm.394530 | procollagen, type VIII, alpha 1 | Col8a1 |
| Mm.21617 | eukaryotic translation initiation factor 2a | Eif2a |
| Mm.333406 | cyclin D2 | Ccnd2 |
| Mm.4618 | trans-acting transcription factor 1 | Sp1 |
| Mm.40828 | p62 / sequestosome 1 | Sqstm1 |
| Mm.4159.1 | thrombospondin 1 | Thsp |

## Claims

1. A host cell deficient for
a) a muscle ring finger 1 (MuRF1) polypeptide, and
b) a muscle ring finger 2 (MuRF2) polypeptide.

2. The host cell of claim 1, wherein the host cell is a mammalian cell.

3. The host cell of claim 2, wherein the mammalian cell is a mouse cell.

4. A non-human organism comprising the host cell of any one of claims 1 to 3.

5. A method for identifying a gene product which is regulated by a MuRF1 polypeptide and a MuRF2 polypeptide, comprising the steps of
a) determining the amount of a gene product suspected to be regulated by the MuRF1 and MuRF2 polypeptide in the host cell of claims 1 to 3 and/or in a sample of the organism of claim 4, and
b) comparing the amount as determined in step a) with a suitable reference amount.

6. The method of claim 5, wherein the reference amount is an amount of the gene product to be tested in a host cell and/or in a sample of an organism which is not deficient for the MuRF1 polypeptide and the MuRF2 polypeptide.

7. The method of claim 5 or 6, wherein a change of the amount as determined in step a) indicates that the tested gene product is regulated by the MuRF1 polypeptide and the MuRF2 polypeptide.

8. A method for identifying a compound capable of influencing expression of a MuRF1 polypeptide and a MuRF2 polypeptide, comprising the steps,
a) administering a test compound to a host cell or a non-human organism expressing the MuRF1 and the MuRF2 polypeptide,
b) determining the amount of the MuRF1 polypeptide and the amount of the MuRF2 polypeptide, and
c) comparing the amounts as determined in step b) with a suitable reference amount,
wherein a change of the amount of the MuRF1 polypeptide and the amount of the MuRF2 polypeptide as determined in step b) compared with the suitable reference amount indicates that the tested compound is capable of influencing expression of the MuRF1 polypeptide and the MuRF2 polypeptide.

9. The method of claim 8, wherein the reference amount is derived from a host cell expressing the MuRF1 polypeptide and the MuRF2 polypeptide to which the compound to be tested has not been administered.

10. The method of claim 8 or 9, wherein a decrease of the amount of the MuRF1 polypeptide and the MuRF2 polypeptide in the host cell or organism to which the compound to be tested was administered compared with the reference amount indicates that the tested compound is capable of inhibiting expression of the MuRF1 polypeptide and the MuRF2 polypeptide.

11. A method for identifying a compound capable of influencing the biological activity of a MuRF1 polypeptide and a MuRF2 polypeptide, comprising the steps,
a) administering a test compound to a host cell or a non-human organism expressing the MuRF1 and the MuRF2 polypeptide,
b) determining the biological activity of the MuRF1 polypeptide and the biological activity of the MuRF2 polypeptide, and
c) comparing the biological activity as determined in step b) with a suitable reference biological activity,
wherein a change of the biological activity of the MuRF1 polypeptide and the biological activity of the MuRF2 polypeptide as determined in step b) compared with the reference biological activity indicates that the tested compound is capable of influencing the biological activity of the MuRF1 polypeptide and the MuRF2 polypeptide.

12. The method of claim 11, wherein the reference biological activity is derived from a host cell comprising the MuRF1 polypeptide and the MuRF2 polypeptide to which the compound to be tested has not been administered.

13. The method of claim 11 or 12, wherein a decrease of the biological activity of the MuRF1 polypeptide and the MuRF2 polypeptide in the host cell or organism to which the compound to be tested was administered compared with the reference amount indicates that the tested compound is capable of inhibiting the biological activity of the MuRF1 polypeptide and the MuRF2 polypeptide.

14. A method for identifying a compound which is capable of influencing the interaction of the MuRF1 and MuRF2 polypeptide and an interaction polypeptide capable of interacting with the MuRF1 and MuRF2 polypeptide, comprising the steps of
a) contacting said MuRF1 polypeptide with said interaction polypeptide and the compound to be tested,
b) contacting said MuRF2 polypeptide with said interaction polypeptide and the compound to be tested,
c) determining the interaction between said MuRF1 polypeptide and said interaction polypeptide,
d) determining the interaction between said MuRF2 polypeptide and said interaction polypeptide,
e) comparing the interaction as determined in step c) with a suitable reference, and
f) comparing the interaction as determined in step d) with a suitable reference
wherein a change of the interaction as determined in step c) and in step d) compared with the corresponding reference indicates that the tested compound influences the interaction between the MuRF1 and MuRF2 polypeptide and said interaction polypeptide.

15. The method of claim 14, wherein a decreased interaction between the MuRF 1 polypeptide and said interaction polypeptide and a decreased interaction between the MuRF2 polypeptide and said interaction polypeptide indicates that the tested compound is capable of inhibiting interaction between the interaction polypeptide and (i) the MuRF1 polypeptide and (ii) the MuRF2 polypeptide.

16. The method of claim 14 or 15, wherein the interaction polypeptide is selected from the group consisting of polypeptides having the following GenBank accession numbers: NP_060828.2, NP_004037.1, NP_001677.2, NP_055206.2, NP_001319.1, AAH07462.1, NP_660287.1, NP_004083.2, NP_001395.1, AAH00734.1, NP_443739.2, NP_004093.1, NP_001433.1, NP_079272.4, AAH07369.2, NP_005317.2, NP_689953.1, NP_055889.2, AAH40035.1, NP_055578.2, NP_872578.1, NP_996557.1, NP_067068.1, AAH69045.1, NP_060912.2, NP_006187.1, NP_114366.1, NP_006784.1, NP_000916.2, NP_002603.1, NP_006654.2, NP_009010.2, NP_060724.1, NP_003720.1, NP_003891.1, AAH09586.1, NP_705582.1, NP_005600.1, NP_004121.2, and NP_000247.2.

17. The method of any one of claims 14 to 16, wherein the interaction is determined by determining the ubiquitination and/or sumoylation of said interaction polypeptide.

18. The method of any one of claims 14 to 16, wherein the interaction is determined by an amplified luminescent proximity homogenous assay.

19. A composition comprising the MuRF1 polypeptide and the MuRF2 polypeptide.

20. A method for identifying a compound capable for the treatment or prevention of diabetes, metabolic syndrome, insulin resistance or chronic heart failure, comprising the steps of
a) administering the compound to be tested to a host cell or organism expressing a MuRF1 polypeptide and/or a MuRF2 polypeptide,
b) determining the amount of the MuRF1 polypeptide and/or the MuRF2 polypeptide, and
c) comparing the amount of the MuRF1 polypeptide and/or the MuRF2 polypeptide as determined in step b) with a suitable reference amount,
wherein a change of the amount of the MuRF1 polypeptide and/or the MuRF2 polypeptide as determined in step b) compared with the reference amount indicates that the tested compound is capable for the treatment or prevention of diabetes, metabolic syndrome, insulin resistance or chronic heart failure.

21. The method of claim 20, wherein the reference amount is derived from a host cell or organism comprising the MuRF1 polypeptide and/or the MuRF2 polypeptide to which the compound to be tested has not been administered, or to which a lower amount of said compound has been administered.

22. The method of claim 21, wherein a decrease of the amount of the MuRF1 polypeptide and/or the MuRF2 polypeptide in the host cell or organism to which the compound to be tested was administered compared with the reference amount for the MuRF1 polypeptide and/or for the MuRF2 polypeptide indicates that said compound is capable for the treatment or prevention of diabetes, metabolic syndrome, insulin resistance or chronic heart failure.

23. A method for identifying a compound capable for the treatment or prevention of diabetes or the myocellular damage caused by diabetes, comprising the steps of
a) administering a compound to be tested to a host cell or organism expressing a MuRF1 polypeptide and/or a MuRF2 polypeptide with,
b) determining the biological activity of the MuRF1 polypeptide and/or the MuRF2 polypeptide, and
c) comparing the biological activity of the MuRF1 polypeptide and/or the MuRF2 polypeptide as determined in step b) with a suitable reference biological activity,
wherein a change of the biological activity of the MuRF1 polypeptide and/or the MuRF2 polypeptide as determined in step b) compared with the reference biological activity indicates that the tested compound is capable for the treatment or prevention of diabetes, or the myocellular damage caused by diabetes.

24. A method for identifying a compound capable for the treatment or prevention of diabetes, metabolic syndrome, insulin resistance or chronic heart failure, comprising the steps of
a) contacting a MuRF1 polypeptide and/or a MuRF2 polypeptide with the compound to be tested and an interaction polypeptide capable of interacting with the MuRF1 and/or MuRF2 polypeptide,
b) determining the interaction between said interaction polypeptide and (i) the MuRF1 polypeptide and/or (ii) the MuRF2 polypeptide,
c) comparing the interaction as determined in step b) with a suitable reference, and
wherein a decrease of the interaction between the interaction polypeptide and the MuRF1 polypeptide or the MuRF1 polypeptide indicates that the tested compound is capable for the treatment or prevention of diabetes, metabolic syndrome, insulin resistance or chronic heart failure.

25. The method of claim 24, wherein the interaction polypeptide is selected from the group consisting of polypeptides having the following GenBank accession numbers: of NP_060828.2, NP_004037.1, NP_001677.2, NP_055206.2, NP_001319.1, AAH07462.1, NP_660287.1, NP_004083.2, NP_001395.1, AAH00734.1, NP_443739.2, NP_004093.1, NP_001433.1, NP_079272.4, AAH07369.2, NP_005317.2, NP_689953.1, NP_055889.2, AAH40035.1, NP_055578.2, NP_872578.1, NP_996557.1, NP_067068.1, AAH69045.1, NP_060912.2, NP_006187.1, NP_114366.1, NP_006784.1, NP_000916.2, NP_002603.1, NP_006654.2, NP_009010.2, NP_060724.1, NP_003720.1, NP_003891.1, AAH09586.1, NP_705582.1, NP_005600.1, NP_004121.2, NP_000247.2. NP_443739.2.


SEQUENCE LISTING

<110>  Labeit, Siegfried

<120>  A host cell deficient for MuRF1 and MuRF2

<130>  LAB65256EP

<160>  15

<170>  PatentIn version 3.4

<210>  1
<211>  340
<212>  PRT
<213>  Homo sapiens

<400>  1

Met Glu Asn Leu Glu Lys Gln Leu Ile Cys Pro Ile Cys Leu Glu Met
1               5                   10                  15

Phe Thr Lys Pro Val Val Ile Leu Pro Cys Gln His Asn Leu Cys Arg
            20                  25                  30

Lys Cys Ala Asn Asp Ile Phe Gln Ala Ala Asn Pro Tyr Trp Thr Ser
        35                  40                  45

Arg Gly Ser Ser Val Ser Met Ser Gly Gly Arg Phe Arg Cys Pro Thr
    50                  55                  60

Cys Arg His Glu Val Ile Met Asp Arg His Gly Val Tyr Gly Leu Gln
65                  70                  75                  80

Arg Asn Leu Leu Val Glu Asn Ile Ile Asp Ile Tyr Lys Gln Glu Cys
                85                  90                  95

Ser Ser Arg Pro Leu Gln Lys Gly Ser His Pro Met Cys Lys Glu His
            100                 105                 110

Glu Asp Glu Lys Ile Asn Ile Tyr Cys Leu Thr Cys Glu Val Pro Thr
        115                 120                 125

Cys Ser Met Cys Lys Val Phe Gly Ile His Lys Ala Cys Glu Val Ala
    130                 135                 140

Pro Leu Gln Ser Val Phe Gln Gly Gln Lys Thr Glu Leu Asn Asn Cys
145                 150                 155                 160

Ile Ser Met Leu Val Ala Gly Asn Asp Arg Val Gln Thr Ile Ile Thr
                165                 170                 175

Gln Leu Glu Asp Ser Arg Arg Val Thr Lys Glu Asn Ser His Gln Val
            180                 185                 190

Lys Glu Glu Leu Ser Gln Lys Phe Asp Thr Leu Tyr Ala Ile Leu Asp
        195                 200                 205

Glu Lys Lys Ser Glu Leu Leu Gln Arg Ile Thr Gln Glu Gln Glu Glu
    210                 215                 220

Lys Leu Ser Phe Ile Glu Ala Leu Ile Gln Gln Tyr Gln Glu Gln Leu
225                 230                 235                 240

Asp Lys Ser Thr Lys Leu Val Glu Thr Ala Ile Gln Ser Leu Asp Glu
                245                 250                 255

Pro Gly Gly Ala Thr Phe Leu Leu Thr Ala Lys Gln Leu Ile Lys Ser
            260                 265                 270

Ile Val Glu Ala Ser Lys Gly Cys Gln Leu Gly Lys Thr Glu Gln Gly
        275                 280                 285

Phe Glu Asn Met Asp Phe Phe Thr Leu Asp Leu Glu His Ile Ala Asp
    290                 295                 300

Ala Leu Arg Ala Ile Asp Phe Gly Thr Asp Glu Glu Glu Glu Glu Phe
305                 310                 315                 320

Ile Glu Glu Glu Asp Gln Glu Glu Glu Glu Ser Thr Glu Gly Lys Glu
                325                 330                 335

Glu Gly His Gln
            340

<210>  2
<211>  436
<212>  PRT
<213>  Homo sapiens

<400>  2

Met Asp Asn Leu Glu Lys Gln Leu Ile Cys Pro Ile Cys Leu Glu Met
1               5                   10                  15

Phe Thr Lys Pro Val Val Ile Leu Pro Cys Gln His Asn Leu Cys Arg
            20                  25                  30

Lys Cys Ala Ser Asp Ile Phe Gln Ala Ser Asn Pro Tyr Leu Pro Thr
        35                  40                  45

Arg Gly Gly Thr Thr Met Ala Ser Gly Gly Arg Phe Arg Cys Pro Ser
    50                  55                  60

Cys Arg His Glu Val Val Leu Asp Arg His Gly Val Tyr Gly Leu Gln
65                  70                  75                  80

Arg Asn Leu Leu Val Glu Asn Ile Ile Asp Ile Tyr Lys Gln Glu Ser
                85                  90                  95

Thr Arg Pro Glu Lys Lys Ser Asp Gln Pro Met Cys Glu Glu His Glu
            100                 105                 110

Glu Glu Arg Ile Asn Ile Tyr Cys Leu Asn Cys Glu Val Pro Thr Cys
        115                 120                 125

Ser Leu Cys Lys Val Phe Gly Ala His Lys Asp Cys Gln Val Ala Pro
    130                 135                 140

Leu Thr His Val Phe Gln Arg Gln Lys Ser Glu Leu Ser Asp Gly Ile
145                 150                 155                 160

Ala Ile Leu Val Gly Ser Asn Asp Arg Val Gln Gly Val Ile Ser Gln
                165                 170                 175

Leu Glu Asp Thr Cys Lys Thr Ile Glu Glu Cys Cys Arg Lys Gln Lys
            180                 185                 190

Gln Glu Leu Cys Glu Lys Phe Asp Tyr Leu Tyr Gly Ile Leu Glu Glu
        195                 200                 205

Arg Lys Asn Glu Met Thr Gln Val Ile Thr Arg Thr Gln Glu Glu Lys
    210                 215                 220

Leu Glu His Val Arg Ala Leu Ile Lys Lys Tyr Ser Asp His Leu Glu
225                 230                 235                 240

Asn Val Ser Lys Leu Val Glu Ser Gly Ile Gln Phe Met Asp Glu Pro
                245                 250                 255

Glu Met Ala Val Phe Leu Gln Asn Ala Lys Thr Leu Leu Lys Lys Ile
            260                 265                 270

Ser Glu Ala Ser Lys Ala Phe Gln Met Glu Lys Ile Glu His Gly Tyr
        275                 280                 285

Glu Asn Met Asn His Phe Thr Val Asn Leu Asn Arg Glu Glu Lys Ile
    290                 295                 300

Ile Arg Glu Ile Asp Phe Tyr Arg Glu Asp Glu Asp Glu Glu Glu Glu
305                 310                 315                 320

Glu Gly Gly Glu Gly Glu Lys Glu Gly Glu Gly Glu Val Gly Gly Glu
                325                 330                 335

Ala Val Glu Val Glu Glu Val Glu Asn Val Gln Thr Glu Phe Pro Gly
            340                 345                 350

Glu Asp Glu Asn Pro Glu Lys Ala Ser Glu Leu Ser Gln Val Glu Leu
        355                 360                 365

Gln Ala Ala Pro Gly Ala Leu Pro Val Ser Ser Pro Glu Pro Pro Pro

370                 375                 380

Ala Leu Pro Pro Ala Ala Asp Ala Pro Val Thr Gln Ile Gly Phe Glu
385                 390                 395                 400

Ala Pro Pro Leu Gln Gly Gln Ala Ala Ala Pro Ala Ser Gly Ser Gly
                405                 410                 415

Ala Asp Ser Glu Pro Ala Arg His Ile Phe Ser Phe Ser Trp Leu Asn
            420                 425                 430

Ser Leu Asn Glu
        435

<210>  3
<211>  2097
<212>  DNA
<213>  Homo sapiens

<400>  3
ttctcaggta ctttatcgga cctctcacat ggctgcatgc ccagaaatgt gatgatattg      60

ttgacagcct cttcaagggt tttggtagaa ctgagggcaa aggtttcctc tttctcaaag     120

gtatctccca cctcttccca agcagcagca aagttaggct gacctcgtct gttatgtaaa     180

ggatgcgtag ggatgggagg gcgatgagga ctaggatgat ggcgggcagg atagttcaga     240

cggtttccat ttcctgagcg tctgagatgt tagtattagt tagttttgtt gtgagtgtta     300

gaattcgggc accaggagaa ggaagccaac aggatccgac ccggtgtttt gtgacaaagg     360

caagaccccc aggtctactt agagcaaagt tagtagagga ggcagctagg cgtggctctc     420

attccttccc acagaatgga ttataagtcg agcctgatcc aggatgggaa tcccatggag     480

aacttggaga agcagctgat ctgccctatc tgcctggaga tgtttaccaa gccagtggtc     540

atcttgccgt gccagcacaa cctgtgccgg aagtgtgcca atgacatctt ccaggctgca     600

aatccctact ggaccagccg gggcagctca gtgtccatgt ctggaggccg tttccgctgc     660

cccacctgcc gccacgaggt gatcatggat cgtcacggag tgtacggcct gcagaggaac     720

ctgctggtgg agaacatcat cgacatctac aaacaggagt gctccagtcg gccgctgcag     780

aagggcagtc accccatgtg caaggagcac gaagatgaga aaatcaacat ctactgtctc     840

acgtgtgagg tgcccacctg ctccatgtgc aaggtgtttg ggatccacaa ggcctgcgag     900

gtggccccat tgcagagtgt cttccaggga caaaagactg aactgaataa ctgtatctcc     960

atgctggtgg cggggaatga ccgtgtgcag accatcatca ctcagctgga ggattcccgt    1020

cgagtgacca aggagaacag tcaccaggta aaggaagagc tgagccagaa gtttgacacg    1080

ttgtatgcca tcctggatga gaagaaaagt gagttgctgc agcggatcac gcaggagcag    1140

gaggaaaagc ttagcttcat cgaggccctc atccagcagt accaggagca gctggacaag    1200

tccacaaagc tggtggaaac tgccatccag tccctggacg agcctggggg agccaccttc    1260

ctcttgactg ccaagcaact catcaaaagc attgtggaag cttccaaggg ctgccagctg    1320

gggaagacag agcagggctt tgagaacatg gacttcttta ctttggattt agagcacata 1380

gcagacgccc tgagagccat tgactttggg acagatgagg aagaggaaga attcattgaa 1440

gaagaagatc aggaagagga agagtccaca gaagggaagg aagaaggaca ccagtaagga 1500

gctggatgaa tgagaggccc ccagatgcag agagactgga gagggtgggg aggggcccag 1560

cggccttggt gacaggccca gggtgggagg ggtcggggcc cctggagggg caatggggag 1620

gtgatgtctt ctctctgctc agagagcagg gactagggta ggaccctcac cgctgcgtcc 1680

agcagacact gaaccagaat tggaaacgtg cttgaaacaa tcacacagga cacttttcta 1740

cattggtgca aaatggaata ttttgtacat ttttaaaatg tgatttttgt atatacttgt 1800

atatgtatgc caatttggtg ctttttgtaa aggaactttt gtataataat gcctggtcgt 1860

tgggtgacct gcgattgtca gaaagagggg aaggaagcca ggttgataca gctgcccact 1920

tcctttcctg agcaggagga tggggtagca ctcacaggga cgatgtgctg tatttcagtg 1980

cctatcccag acatacgggg tggtaactga gtttgtgtta tatgttgttt taataaatgc 2040

acaatgctct cttcctgttc ttcaaaaaaa aaaaaaaaaa aaaaaaaaaa aaaaaaa 2097

<210> 4
<211> 2202
<212> DNA
<213> Homo sapiens

<400> 4
aaacaatgtc ctccaccgag agaaacgtaa aggacacttg atcacacaat ccctggaata 60

atatccagga aacacttgct ggagccactc gcagcaccct tccctggcag cacacttggg 120

gacagcgagg agatgagcgc atctctgaat tacaaatctt tttccaaaga gcagcagacc 180

atggataact tagagaagca actcatctgt cccatctgct tagagatgtt cacgaaacct 240

gtggtgattc tcccttgtca gcacaacctg tgtaggaaat gtgccagtga tattttccag 300

gcctctaacc cgtatttgcc cacaagagga ggtaccacca tggcatcagg gggccgattc 360

cgctgcccat cctgtagaca tgaagtggtt ttggatagac atggggtata tggacttcag 420

aggaacctgc tggtggaaaa tatcattgac atctacaagc aggagtccac caggccagaa 480

aagaaatccg accagcccat gtgcgaggaa catgaagagg agcgcatcaa catctactgt 540

ctgaactgcg aagtacccac ctgctctctg tgcaaggtgt ttggtgcaca caaagactgc 600

caggtggctc ccctcactca tgtgttccag agacagaagt ctgagctcag tgatggcatc 660

gccatcctcg tgggcagcaa cgatcgagtc cagggagtga tcagccagct ggaagacacc 720

tgcaaaacta tcgaggaatg ttgcagaaaa cagaaacaag agctttgtga gaagtttgat 780

tacctgtatg gcattttgga ggagaggaag aatgaaatga cccaagtcat tacccgaacc 840

caagaggaga aactggaaca tgtccgtgct ctgatcaaaa agtattctga tcatttggag 900

aacgtctcaa agttggttga gtcaggaatt cagtttatgg atgagccaga aatggcagtg 960

tttctgcaga atgccaaaac cctgctaaaa aaaatctcgg aagcatcaaa ggcatttcag 1020

atggagaaaa tagaacatgg ctatgagaac atgaaccact tcacagtcaa cctcaataga 1080

gaagaaaaga taatacgtga aattgacttt tacagagaag atgaagatga agaagaagaa 1140

gaaggcggag aaggagaaaa agaaggagaa ggagaagtgg gaggagaagc agtagaagtg 1200

gaagaggtag aaaatgttca aacagagttt ccaggagaag atgaaaaccc agaaaaagct 1260

tcagagctct ctcaggtgga gctgcaggct gcccctgggg cacttccagt ttcctctcca 1320

gagccacctc cagccctgcc acctgctgcg gatgcccctg tgacacaggg ggaggttgta 1380

cccactggct ctgagcagac cacagagtct gaaactccag tccctgcagc agcagaaact 1440

gcggatccct tgttttaccc tagttggtat aaaggccaaa cccggaaagc caccaccaac 1500

ccaccttgca ccccagggag cgaaggtctg gggcaaatag ggcctccagg ttctgaggat 1560

tcgaatgtac ggaaggcaga agtggcagca gccgcagcga gtgagagggc agctgtgagt 1620

ggtaaggaaa ctagtgcacc tgcagctact tctcagattg gatttgaggc tcctcccctc 1680

cagggacagg ctgcagctcc agcgagtggc agtggagctg attctgagcc agctcgccat 1740

atcttctcct tttcctggtt gaactcccta aatgaatgat attcattcca actgctgccc 1800

ctctgtctgc ctggctgaga tgcatgtggg cagcaggaag cccaagtgaa attaatatta 1860

tgcagatgat gaaagggacc tctgaacagg atttctgcaa aaatagcccc aaactgcaat 1920

tccatatgac ttatctaaca tcttgggggg aaagaatatt ttgagaaaat agttgcagaa 1980

agcactggaa ataataaact tgatcttata caaatcttct attgtgtgga aaatgttgtg 2040

aagggtgtgt aggtgtggta catgtgtatg tcactaacaa gtggcaaatg gtgaaaaaag 2100

tggtcactat gcttttgtct ctcataggca ctgacttttt gttattatat tatggtagct 2160

ttcatttcct ttactcttta acagtgcagg tggtcagtga aa 2202

<210> 5
<211> 212
<212> PRT
<213> Homo sapiens

<400> 5

Lys Ala Ser His Pro Met Cys Lys Glu His Glu Asp Glu Lys Ile Asn
1               5                   10                  15

Ile Tyr Cys Leu Thr Cys Glu Val Pro Thr Cys Ser Met Cys Lys Val
            20                  25                  30

Phe Gly Ile His Lys Ala Cys Glu Val Ala Pro Leu Gln Ser Val Phe
        35                  40                  45

Gln Gly Gln Lys Thr Glu Leu Asn Asn Cys Ile Ser Met Leu Val Ala
    50                  55                  60

Gly Asn Asp Arg Val Gln Thr Ile Ile Thr Gln Leu Glu Asp Ser Arg
65                  70                  75                  80

Arg Val Thr Lys Glu Asn Ser His Gln Val Lys Glu Glu Leu Ser Gln
                85                  90                  95

Lys Phe Asp Thr Leu Tyr Ala Ile Leu Asp Glu Lys Lys Ser Glu Leu
            100                 105                 110

Leu Gln Arg Ile Thr Gln Glu Gln Glu Glu Lys Leu Ser Phe Ile Glu
        115                 120                 125

Ala Leu Ile Gln Gln Tyr Gln Glu Gln Leu Asp Lys Ser Thr Lys Leu
    130                 135                 140

Val Glu Thr Ala Ile Gln Ser Leu Asp Glu Pro Gly Gly Ala Thr Phe
145                 150                 155                 160

Leu Leu Thr Ala Lys Gln Leu Ile Lys Ser Ile Val Glu Ala Ser Lys
                165                 170                 175

Gly Cys Gln Leu Gly Lys Thr Glu Gln Gly Phe Glu Asn Met Asp Phe
            180                 185                 190

Phe Thr Leu Asp Leu Glu His Ile Ala Asp Ala Leu Arg Ala Ile Asp
        195                 200                 205

Phe Gly Thr Asp
    210

<210>  6
<211>  212
<212>  PRT
<213>  Homo sapiens

<400>  6

Lys Ser Asp Gln Pro Met Cys Glu Glu His Glu Glu Glu Arg Ile Asn
1               5                   10                  15

Ile Tyr Cys Leu Asn Cys Glu Val Pro Thr Cys Ser Leu Cys Lys Val
            20                  25                  30

Phe Gly Ala His Lys Asp Cys Gln Val Ala Pro Leu Thr His Val Phe
        35                  40                  45

Gln Arg Gln Lys Cys Glu Leu Ser Asp Gly Ile Ala Ile Leu Val Gly
    50                  55                  60

Ser Asn Asp Arg Val Gln Gly Val Ile Ser Gln Leu Glu Asp Thr Cys
65                  70                  75                  80

Lys Thr Ile Glu Glu Cys Cys Arg Lys Gln Lys Gln Glu Leu Cys Glu
                85                  90                  95

Lys Phe Asp Tyr Leu Tyr Gly Ile Leu Glu Glu Lys Arg Asn Glu Met
            100                 105                 110

Thr Gln Val Ile Thr Arg Thr Gln Glu Glu Lys Leu Glu His Val Arg

115                 120                 125

Ala Leu Ile Lys Lys Tyr Ser Asp His Leu Glu Asn Val Ser Lys Leu
    130                 135                 140

Val Glu Ser Gly Ile Gln Phe Met Asp Glu Pro Glu Met Ala Val Phe
145                 150                 155                 160

Leu Gln Asn Ala Lys Thr Leu Leu Lys Lys Ile Ser Glu Ala Ser Lys
                165                 170                 175

Ala Phe Gln Met Glu Lys Ile Glu His Gly Tyr Glu Asn Met Asn His
            180                 185                 190

Phe Thr Val Asn Leu Asn Arg Glu Glu Lys Ile Ile Arg Glu Ile Asp
        195                 200                 205

Phe Tyr Arg Glu
    210

<210>  7
<211>  22
<212>  DNA
<213>  artificial

<220>
<223>  MuRF1 forward primer

<400>  7
agaagtcggg ggtcagggga cg                                              22

<210>  8
<211>  30
<212>  DNA
<213>  artificial

<220>
<223>  MuRF1 reverse primer

<400>  8
ggtccatgat cacttcatgg cggcacgagg                                      30

<210>  9
<211>  31
<212>  DNA
<213>  artificial

<220>
<223>  MuRF2 forward primer

<400>  9
gagcacttct ctgaattaca agtctttctc c                                    31

<210>  10
<211>  28
<212>  DNA
<213>  artificial

<220>

<223>  MuRF2 reverse primer

<400>  10
gctgagagcc aatgggcacc acctcccc                                          28


<210>  11
<211>  31
<212>  DNA
<213>  artificial

<220>
<223>  MuRF3 forward primer

<400>  11
gccccatctg cctggagatg ttctccaagc c                                      31


<210>  12
<211>  29
<212>  DNA
<213>  artificial

<220>
<223>  MuRF3 reverse primer

<400>  12
gccgctcctc ctccagcccc gcattgccc                                         29


<210>  13
<211>  22
<212>  DNA
<213>  artificial

<220>
<223>  Aldolase forward primer

<400>  13
agctgtctga catcgctcac cg                                                22


<210>  14
<211>  22
<212>  DNA
<213>  artificial

<220>
<223>  Aldolase reverse primer

<400>  14
cacatactgg cagcgcttca ag                                                22


<210>  15
<211>  20
<212>  DNA
<213>  artificial

<220>
<223>  MuRF1/MuRF2 RNAi target

<400>  15
cagaggaacc tgctggtgga                                                   20